(19) Europäisches Patentamt
European Patent Office
Office européen des brevets

(11) EP 1 704 849 A1

(12) # EUROPEAN PATENT APPLICATION
published in accordance with Art. 158(3) EPC

(43) Date of publication:
**27.09.2006 Bulletin 2006/39**

(21) Application number: **05703571.9**

(22) Date of filing: **14.01.2005**

(51) Int Cl.:
***A61K 8/18*** *(2006.01)* ***C08L 75/04*** *(2006.01)*
***C08G 18/66*** *(2006.01)*

(86) International application number:
**PCT/JP2005/000332**

(87) International publication number:
**WO 2005/067884 (28.07.2005 Gazette 2005/30)**

(84) Designated Contracting States:
**DE FR IT**

(30) Priority: **14.01.2004 JP 2004006285**
**25.08.2004 JP 2004245079**

(71) Applicant: **SHISEIDO COMPANY, LTD.**
**Tokyo 104-8010 (JP)**

(72) Inventors:
- **OMURA, Takayuki,**
**c/o SHISEIDO RESEARCH CENTER**
**Yokohama-shi Kanagawa 2248558 (JP)**
- **WATANABE, Hiroko,**
**c/o SHISEIDO RESEARCH CENTER**
**Yokohama-shi Kanagawa 2248558 (JP)**

(74) Representative: **Merkle, Gebhard et al**
**TER MEER STEINMEISTER & PARTNER GbR,**
**Patentanwälte,**
**Mauerkircherstrasse 45**
**81679 München (DE)**

(54) **SKIN PREPARATIONS FOR EXTERNAL USE FOR WRINKLE DIMINUTION**

(57) To provide a skin treatment composition for wrinkle reduction that is superior in terms of the wrinkle reduction effect, usability, and the sensation during use.

A skin treatment composition having a water dispersion of a polymer in which a non-water soluble film-forming polymer is dispersed in water wherein the main ingredients of said film-forming polymer are polyurethane having a film shrinkage rate of 20% or less and an acrylic type polymer having a film shrinkage rate of 20% or less.

Also, a skin treatment composition for wrinkle reduction comprising (a) a non-emulsification type cross-linked silicone, (b) a film forming polymer having a film shrinkage rate of 20% or less containing as a main ingredient a polyurethane having a film shrinkage rate of 20%, (c) a liquid oil component, and (d) water.

EP 1 704 849 A1

## Description

TECHNICAL FIELD

The present invention relates to a skin treatment composition for wrinkle reduction. More specifically, it relates to a skin treatment composition that reduces skin wrinkles by means of a film formed as said skin treatment composition dries after being applied on the skin.

BACKGROUND ART

Conventionally, film agents have been used for a skin treatment composition for the purpose of reducing skin wrinkles. A film agent is used because the film shrinks as it dries; the idea is to pull small skin wrinkles with the film-forming contractile force to increase the tension and thus temporarily remove the small wrinkles. For this reason, film agents that have a strong contractile force and form a hard film have been used.

Known film agents that are blended into a wrinkle reducing agent using such strongly contracting polymers include, for example, polyurethane (see Patent Document 1); also, research has been done to study wrinkle reduction by means of formation of a film having a strong contractile force by using film-forming polymers such as acrylic resins, vinyl acetate resins, polyethylene resins, silicone resins, polyvinyl resins, polyvinyl alcohol, acrylic water soluble resins, cellulose water soluble resins, starch and its derivatives, gelatin, and sodium alginate (see Patent Document 2, for example).

However, when a film agent having a strong contractile force is used, there are problems in that the wrinkle reduction effect is very limited and usability and the sensation during use are poor; this is because as the film agent contracts its adhesion to the skin weakens and it peels off easily from the skin, which is more elastic, and also because hardness of the film causes discomfort during use, premature breakage of the film, and glossy skin.

Patent Document 1: Japanese Patent Laid-Open H11-504949 bulletin
Patent Document 2: Japanese Patent Laid-Open H5-933 bulletin

DISCLOSURE OF INVENTION

[Problem that the present invention aims to solve]

The present invention has been carried out in view of the situation described above, and its object is to provide a skin treatment composition for wrinkle reduction that is superior in terms of the wrinkle reduction effect, usability, and the sensation during use.

[Means to solve the Problem]

[Invention of claims 1-12]

The inventors conducted earnest research and discovered that the aforementioned problems can be solved by preparing a skin treatment composition by blending a water dispersion of a specific polymer, specifically polyurethane and an acrylic type polymer, that forms a film having a weak contractile force, thus completing the present invention.

The present invention is not based on the idea of using a conventional contracting film; on the contrary, it is based on the idea of 'filling wrinkles with film.' That is, polyurethane and acrylic type polymers are used to obtain a new skin treatment composition for wrinkle reduction having superior functions than those conventionally known using an action mechanism different from those conventionally known: the skin treatment composition for wrinkle reduction of the present invention is applied on the skin to form a film on the skin that does not contract or become hard like the conventional ones.

That is, the present invention is a skin treatment composition having a water dispersion of a polymer in which a non-water soluble film-forming polymer is dispersed in water wherein the main ingredients of said film-forming polymer are polyurethane having a film shrinkage rate of 20% or less and an acrylic type polymer having a film shrinkage rate of 20% or less.

Said film-forming polymer is a polymer that forms a film on the skin or substrate when a water dispersion of it is applied on the skin or substrate and dried.

Said polyurethane is preferably polyurethane obtained by reacting an isocyanate compound and a diol compound containing a polyether diol, polycarbonate diol, and alkylene diol containing a carboxyl group; for said isocyanate compound it is preferable to contain isophorone diisocyanate.

Furthermore, in said preferable polyurethane, the polyether diol is preferably polytetramethylene glycol, the

polycarbonate diol is preferably polyhexamethylene carbonate diol, and the alkylene diol containing a carboxyl group is preferably dimethylolpropionic acid and/or dimethylolbutanoic acid.

Said acrylic type polymer is preferably a polymer of monomers whose main ingredient is ethyl acrylate.

Furthermore, it is preferable for the water dispersion of the acrylic type polymer to contain sulfonated polyvinyl alcohol.

Also, in the present invention, the film strength of said polyurethane is preferably 300-700 kg/cm$^2$, and the film strength of the acrylic type polymer is preferably 0.1-100 kg/cm$^2$.

The film elongation of the polyurethane is preferably 200-500%, and the film elongation of the acrylic type polymer is preferably 500-2000%.

Also, the average particle size of the polyurethane in said water dispersion of polyurethane is preferably 10-300 nm, and the average particle size of the acrylic type polymer in said water dispersion of acrylic type polymer is preferably 100-600 nm.

In the present invention, the polyurethane in the water dispersion of polyurethane preferably is a mixture of particles having an average particle size of 20-60 nm and particles having an average particle size of 150-200 nm.

The skin treatment composition for wrinkle reduction of the present invention preferably contains the polyurethane to 1-10 wt% of the total weight of the skin treatment composition and the acrylic type polymer to 1-20 wt% of the total weight of the skin treatment composition.

The present invention can also contain scaly silica. By adding scaly silica, the film formed by the water dispersion of the polymer becomes thicker and the wrinkle reduction effect is augmented and reinforced and thus a skin treatment composition for wrinkle reduction that can accommodate the elasticity and movement of the skin for a long duration of time can be obtained.

The contractile force in the present invention is a force with which the water dispersion of the polymer contracts when it forms a film, and this force is evaluated based on the film shrinkage rate. The film shrinkage rate is a degree of contraction of the film relative to the thin layer of the original water dispersion when said thin layer of the polymer water dispersion is dried to form the film. Specifically, a water dispersion of a solid equivalent 1 g of the polymer is poured into a 5 cm x 5 cm polyethylene mold such that the film thickness is approximately 0.5 mm and dried at a prescribed temperature (50°C) for a prescribed amount of time (three days at room temperature) to obtain a film, and the vertical height and horizontal length of this film are measured and used in the following formula for calculation.

$$\text{Film shrinkage rate (\%)} = [(\text{Vertical measurement} \times \text{Horizontal measurement})/25] \times 100$$

The film strength and the film elongation of the polymer is the strength and elongation of the film obtained by drying the thin layer of the water dispersion of the polymer; specifically, a water dispersion of a solid equivalent 1 g of the polymer is poured into a 5 cm x 5 cm polyethylene mold such that the film thickness is approximately 0.5 mm and dried at a prescribed temperature (50°C) for a prescribed amount of time (three days at room temperature) to obtain a film, and this film is cut out using a dumbbell #3 and the autograph function of "Tensile tester RTM-250" from Orientec Co., Ltd. is used to carry out the measurement at 20°C and a cross head speed of 300 mm/min.

[Invention of claims 13-25]

The inventors conducted earnest research to solve the aforementioned problems and discovered that the aforementioned problems can be solved by preparing a skin treatment composition by blending in a non-emulsifying type cross-linked silicone and polyurethane that forms a film having a weak contractile force, thus completing the present invention.

The present invention is not based on the idea of using a conventional contracting film; on the contrary, it is based on the idea of using a paste-like material that can change its form freely according to the wrinkle site and non-contracting film, thus 'filling wrinkles with film'. That is, a non-emulsifying type free-form cross-linked silicone and polyurethane, as a non-contracting film agent, are used to obtain a new skin treatment composition for wrinkle reduction having superior functions than those conventionally known using an action mechanism different from those conventionally known.

That is, the present invention is a skin treatment composition for wrinkle reduction comprising (a) a non-emulsification type cross-linked silicone, (b) a film forming polymer having a film shrinkage rate of 20% or less containing as a main ingredient a polyurethane having a film shrinkage rate of 20%, (c) a liquid oil component, and (d) water.

Said film-forming polymer is a polymer that forms a film on the skin or substrate when a water dispersion of it is applied on the skin or substrate and dried.

Said non-emulsification type cross-linked silicone is preferably one, two, or more chosen from a group consisting of a cross polymer derived from a reaction between methyl hydrogen polysiloxane and methyl vinyl polysiloxane, a cross polymer derived from a reaction between a partial long chain alkylated methyl hydrogen polysiloxane and methyl vinyl polysiloxane, and a cross polymer derived from a reaction between methyl hydrogen polysiloxane and alkene.

The long chain alkyl of said partial long chain aklylated methyl hydrogen polysiloxane should preferably have 10-14 carbon atoms.

The non-emulsifying type cross-linked silicone should preferably be added as it is swollen with the liquid oil component.

Said polyurethane is preferably polyurethane obtained by reacting an isocyanate compound and a diol compound containing a polyether diol, polycarbonate diol, and alkylene diol containing a carboxyl group; said isocyanate compound preferably contains isophorone diisocyanate.

Furthermore, in said preferable polyurethane, the polyether diol is preferably polytetramethylene glycol, the polycarbonate diol is preferably polyhexamethylene carbonate diol, and the alkylene diol containing a carboxyl group is preferably dimethylolpropionic acid and/or dimethylolbutanoic acid.

Also, in the present invention, the film strength of said polyurethane is preferably 300-700 kg/cm$^2$.

The film elongation of the polyurethane is preferably 200-500%.

Said film forming polymer having a film shrinkage rate of 20% or less containing as a main ingredient a polyurethane having a film shrinkage rate of 20% is preferably added in the form of a water dispersion.

The average particle size of the polyurethane in said water dispersion of polyurethane is preferably 10-300 nm.

In the present invention, the polyurethane in said water dispersion of polyurethane is preferably a mixture of particles having an average particle size of 20-60 nm and particles having an average particle size of 150-200 nm.

The skin treatment composition for wrinkle reduction of the present invention preferably contains the non-emulsifying type cross-linked silicone to 0.5-5.0 wt% of the total amount of the skin treatment composition, and the polyurethane having a film shrinkage rate of 20% or less to 0.1-10.0 wt% of the total amount of the skin treatment composition.

The contractile force in the present invention is a force with which the water dispersion of the polymer contracts when it forms a film, and this force is evaluated based on the film shrinkage rate. The film shrinkage rate is a degree of contraction of the film relative to the thin layer of the original water dispersion when said thin layer of the polymer water dispersion is dried to form the film. Specifically, a water dispersion of a solid equivalent 1 g of the polymer is poured into a 5 cm x 5 cm polyethylene mold such that the film thickness is approximately 0.5 mm and dried at a prescribed temperature (50˚C) for a prescribed amount of time (three days at room temperature) to obtain a film, and the vertical height and horizontal length of this film are measured and used in the following formula for calculation.

$$\text{Film shrinkage rate (\%)} = [(\text{Vertical measurement} \times \text{Horizontal measurement})/25] \times 100$$

The film strength and the film elongation of the polymer is a strength and elongation of the film obtained by drying a thin layer of the water dispersion of the polymer; specifically, a water dispersion of a solid equivalent 1 g of the polymer is poured into a 5 cm x 5 cm polyethylene mold such that the film thickness is approximately 0.5 mm and dried at a prescribed temperature (50˚C) for a prescribed amount of time (three days at room temperature) to obtain a film, and this film is cut out using a dumbbell #3 and the autograph function of "Tensile tester RTM-250" from Orientec Co., Ltd. is used to carry out the measurement at 20˚C and a cross head speed of 300 mm/min.

[Effects of the invention]

The skin treatment composition for wrinkle reduction of the present invention is not sticky and at the same time soft, supple, elastic, and flexible; it does not crack or peel off; it adheres well to the skin and gives a light tactile sensation without discomfort; it can be worn for a long duration of time with ease and maintain a state of removing even small wrinkles for a long duration of time (i.e. the wrinkle reduction effect lasts for a long duration of time); it therefore exhibits a wrinkle reduction effect that hasn't been known conventionally.

Furthermore, this effect is augmented and reinforced by adding scaly silica, resulting in an exceptional wrinkle reduction effect. In particular, the film formed by the water dispersion of the polymer becomes thick and the wrinkle

reduction effect is augmented and reinforced and thus a skin treatment composition for wrinkle reduction that can accommodate the elasticity and movement of the skin for a long duration of time can be obtained.

BEST MODE FOR CARRYING OUT THE INVENTION

[Invention of claims 1-12]

The best embodiments of the present invention are described in detail below.

The skin treatment composition for wrinkle reduction of the present invention contains a water dispersion of a polymer in which a water-insoluble film forming polymer is dispersed in water, and the main ingredients of said film forming polymer are polyurethane having a film shrinkage rate of 20% or less and an acrylic type polymer having a film shrinkage rate of 20% or less. The film shrinkage rate is defined as described above.

Those for which said film shrinkage rate is over 20% have a poor wrinkle reduction effect, peel off the skin, cause noticeable "gloss", and exhibit poor usability due to discomfort; therefore they cannot manifest the effects of the present invention. The preferable range of the film shrinkage rate is 10% or less. The lower limit of the film shrinkage rate is 0, i.e. no shrinkage, which is preferable; however, 5% is sufficient and also practical. That is, the film shrinkage rate in the present invention is 0-20%; within this range, preferable combinations of the upper and lower limits are used, examples include 5-20%, 0-10%, and 5-10%.

In the present invention, as described above, a water dispersion of polyurethane, in which non-water-soluble polyurethane is dispersed in water, is used; said water dispersion is applied on the skin or substrate and as it is dried a polyurethane film is formed on the skin or substrate.

The polyurethane used in the present invention is a polymer having urethane bonds; the urethane bond is formed by an addition reaction between an isocyanate group and a compound having active hydrogen, such as a hydroxyl group. The polyurethane in the present invention is preferably obtained with a conventional method reacting at least (A) an isocyanate compound having two isocyanate groups and (B) a diol compound having two hydroxyl groups.

Selection of said isocyanate compound (ingredient A) is not limited in particular as long as it is used in conventional polyurethane manufacturing, examples include organic diisocyanate compounds such as aliphatic diisocyanate compounds, alicyclic isocyanate compounds, and aromatic diisocyanate compounds. More preferable are aliphatic diisocyanate compounds and alicyclic diisocyanate compounds. 0ne, two or more isocyanate compounds are freely selected and used.

Examples of said aliphatic diisocyanate compounds include ethylene diisocyanate, 2,2,4-trimethylhexamethylene diisocyanate, and 1,6-hexamethylene diisocyanate.

Examples of said alicyclic diisocyanate compounds include hydrogenated 4,4'-diphenylmethane diisocyanate, 1,4-cyclohexane diisocyanate, methylcyclohexylene diisocyanate, isophorone diisocyanate (hereafter referred to as "IPDI"), and norbornane diisocyanate.

Examples of said aromatic diisocyanate compound include 4,4'-diphenylmethane diisocyanate, xylylene diisocyanate, toluene diisocyanate, and naphthalene diisocyanate.

Of the specific examples of said isocyanate compound (ingredient A), 1,6-hexymethylene diisocyanate, IPDI, and norbornane diisocyanate are preferable due to superior weather resistance and availability. IPDI is particularly preferable.

Selection of said diol compound (ingredient B) is not limited in particular as long as it is used in conventional polyurethane manufacturing, preferable examples include an alkylene diol, carboxyl group-containing alkyl diol, alicyclic diol, spiro diol, polyester diol, polyether diol, polycarbonate diol, polybutadiene diol, polyisoprene diol, and polyolefin diol. Of these, particularly preferably used are an alkylene diol, carboxyl group-containing alkylene diol, alicyclic diol, polyether diol, and polycarbonate diol. One, two or more diol compounds are freely selected and used.

Examples of said alkylene diol include ethylene glycol, propylene glycol, 1,4-butane diol, 1,6-hexane diol, neopentyl glycol, 1,8-octane diol, and 1,10-decane diol.

Preferable examples of said carboxyl group-containing alkylene diol include carboxylic acids having 3-26 carbons, more preferably 3-12 carbons, as well as having a dialylol groups, such as dimethylol, diethanol and propanol. Specific examples include dimethylol propionic acid (hereafter "DMPA") and dimethylol butanoic acid (hereafter "DMBA"); a mixture thereof can be used as well.

Examples of said alicyclic diol include 1,4-cyclohexane dimethanol (hereafter "CHDM"), which is preferable because it gives adequate strength to the film.

Preferable examples of said spiro diol include spiro glycol.

Examples of said polyester diol include those obtained by condensation polymerization between at least one chosen from a group of dicarboxylic acids including succinic acid, glutaric acid, adipic acid, sebacic acid, azelaic acid, maleic acid, fumaric acid, phthalic acid, and terephthalic acid and at least one chosen from a group of diols including ethylene glycol, propylene glycol, 1,4-butane diol, 1,6-hexane diol, neopentyl glycol, 1,8-octane diol, 1,10-decane diol,

diethylene glycol, polyethylene glycol (hereafter "PEG"), polypropylene glycol (hereafter "PPG"), tetramethylene glycol, polytetramethylene glycol, and spiro glycol, as well as those obtained by the ring opening polymerization of lactonic acid.

Examples of said polyether diol include polyether diol contained in diol used in synthesis of said polyester diol, examples of which include a polyoxyethylene diol such as diethylene glycol and PEG, a polyoxypropylene diol such as PPG, a polyoxytetramethylene diol such as polytetramethylene glycol (hereafter "PTMG"), phenols such as bisphenol A, and a product of the ring opening addition polymerization of bisphenol A and at least one compound chosen from propylene oxide (hereafter "P0") and ethylene oxide (hereafter "E0") (if a copolymer is used, such a copolymer can be either a block copolymer or random copolymer). Of these, PEG, PPG, and PTMG are preferable, and PTMG is particularly preferable since it forms a soft film and adheres to the skin very well.

Preferable examples of said polycarbonate diol include polyhexamethylene carbonate diol (hereafter "PHMC") because of its ability to form a soft film and its superior adhesion to the skin.

In the present invention, polyurethane that forms a superior film can be obtained by using the diol compound (ingredient B) consisting of a mixture of a polyether diol, polycarbonate diol, and carboxyl group-containing alkylene diol.

In the present invention, when a carboxyl group-containing alkylene diol is used for the diol compound in the synthesis to obtain polyurethane containing carboxyl groups in the molecule, the carboxyl groups incorporated in the molecule can be neutralized by neutralizers such as triethylamine, trimethylamine, 2-amino-2-methyl-1-propanol, triethanolamine, potassium hydroxide, and sodium hydroxide to obtain a stable water dispersion of polyurethane.

In the present invention, when mixing a polyether diol, polycarbonate diol, and carboxyl group-containing alkylene diol, it is particularly preferable to use PTMG for said polyether diol, PHMC for the polycarbonate diol, and DMPA and/or DMBA for said carboxyl group-containing alkylene diol.

In the present invention, when using these preferable diol compounds, it is particularly preferable to use an isocyanate compound that contains IPDI; a skin treatment composition for wrinkle reduction having an exceptional wrinkle reduction effect can be obtained by using polyurethane synthesized by using IPDI for the isocyanate and PTMG, PHMC, DMPA and/or DMBA for the diol compounds.

The molar ratio of said isocyanate compound (ingredient A) and diol compound (ingredient B) is preferably A/B = 2/0.8-2/1.8, and more preferably A/B = 2/1-2/1.8.

In the present invention, it is preferable to use polyurethane having structural units derived from alkylene oxide (R0); this makes it easier to control the elongation of the film obtained from the water dispersion of the polyurethane to obtain a flexible film. A skin treatment composition that manifests superior effects and superior usability can be obtained by using this polyurethane for the preparation.

Examples of the compound having a structural unit derived from R0 include a polyoxyethylene diol such as diethylene glycol and PEG, a polyoxypropylene diol such as PPG, a polyoxytetramethylene diol such as PTMG, polyoxyethylene polyoxypropylene glycol (E0/P0 block copolymer), phenols such as bisphenol A, and a product of the ring opening addition polymerization of bisphenol A and at least one compound chosen from P0 and E0 (if a copolymer is used, such a copolymer can be either a block copolymer or random copolymer); preferably used are PEG, PPG, PTMG, etc.

In the present invention, the compound having a structural unit derived from R0 is used as a polyether diol ingredient in said ingredient B.

The water dispersion of polyurethane is prepared with a conventional method; for example, a pre-polymer having remaining isocyanate obtained from a reaction in an organic solvent is dispersed in water containing potassium hydroxide under high speed agitation, followed by a chain elongation reaction to increase the molecular weight, and said organic solvent is recovered from the obtained water based solution to obtain the water dispersion of the polyurethane.

In the present invention, the following is particularly preferable for said polyurethane and water dispersion of polyurethane. That is:

With regard to the film characteristics of the polyurethane, the strength is 300-700 $kg/cm^2$, and preferably 400-600 $kg/cm^2$. If the strength is less than 300 $kg/cm^2$, then the film cannot follow the skin movements and peeling easily occurs. If it is over 700 $kg/cm^2$, then the film causes discomfort on the skin. The elongation is 200-500%, preferably 300-500%. If the elongation is less than 200%, then the film formed on the skin cannot follow the skin movements very well. If it is over 500%, then the wrinkle reduction effect becomes insufficient.

The average particle size of the polyurethane in the water dispersion of the polyurethane is 10-300 nm, preferably 20-200 nm. If the average particle size is less than 10 nm, then there is a sufficient small-wrinkle reduction effect but on the other hand the large-wrinkle reduction effect becomes insufficient. If the average particle size is over 300 nm, then adhesion to the skin becomes poor and peeling tends to occur.

A preferable embodiment in the present invention uses two kinds of water dispersions of polyurethane having different particle sizes. This way, not only the wrinkle reduction effect but also a skin mark erasing effect can be obtained. For said two kinds of polyurethane having different particle sizes, polyurethanes having an average particle size of 20-60 nm and an average particle size of 150-200 nm are preferable.

The blend ratio of the polyurethane is preferably 0.1-10 wt% of the total amount of the skin treatment composition. If the blend ratio is less than 0.1 wt% then the effect of the present invention cannot be obtained sufficiently; and if it is over 10 wt% then the film tends to peel off the skin. A more preferable blend ratio range is 1-8 wt% of the total amount of the skin treatment composition.

Also, in the present invention, as described above, a water dispersion of an acrylic type polymer, i.e. a water-insoluble acrylic type polymer is dispersed in water, is used; said water dispersion is applied on the skin or substrate and upon drying forms a acrylic type polymer film on the skin or substrate. For said water dispersion, it is preferable to use those conventionally known as polymer emulsions such as a polymer emulsion obtained by emulsification polymerization of acrylic type monomers. Said acrylic type polymer has so-called film forming properties, which means, when the polymer emulsion is applied on the skin or substrate, it forms a film on the skin or substrate as it dries. Therefore, although in the present invention the acrylic type polymer emulsion is preferably dispersed, i.e. diluted, in water before blending, it is also acceptable to use the acrylic type polymer emulsion as is for the water dispersion.

For the acrylic type polymer of the present invention, polymers derived from monomers containing acrylic ester type monomers such as acrylic ester and methacrylic ester are preferable. Specific examples of said acrylic ester type monomers include methyl acrylate, ethyl acrylate, propyl acrylate, n-butyl acrylate, tert-butyl acrylate, hexyl acrylate, cyclohexyl acrylate, octyl acrylate, 2-ethylhexyl acrylate, methyl methacrylate, ethyl methacrylate, propyl methacrylate, n-butyl methacrylate, tert-butyl methacrylate, hexyl methacrylate, cyclohexyl methacrylate, octyl methacrylate, and 2-ethylhexyl methacrylate. The acrylic ester type monomers are used individually or in combinations of two or more.

The monomers for making said acrylic type polymer can contain, in addition to the acrylic ester type monomers, other hydrophobic monomers. Examples of said hydrophobic monomers include aromatic mono- and di- vinyl compounds such as styrene, $\alpha$-styrene, chlorostyrene, alkyl styrene, and divinyl styrene, vinyl cyanide compounds such as acrylonitrile and methacrylonitrile, vinyl esters such as vinyl acetate, vinyl halogenate such as vinyl chloride and vinylidene chloride, fluorocarbon monomers such as trifluoroethyl methacrylate, 2, 2, 3, 3-tetrafluoropropyl methacrylate, 2, 2, 3, 3, 4, 4-hexafluorobutyl methacrylate, perfluorooctyl methacrylate, and perfluorooctyl acrylate, and silicone macro monomers.

It is common to copolymerize the acrylic type polymers of the present invention by further adding hydrophilic monomers. This way, stable polymer emulsion, i. e. stable water dispersion of an acrylic type polymer, can be obtained.

Examples of the hydrophilic monomers include ethylene type unsaturated carboxylic acids such as acrylic acid, methacrylic acid, itaconic acid, maleic acid, fumaric acid, and crotonic acid, ethylene type monomers containing a hydroxyl group such as hydroxyethyl acrylate, hydroxyethyl methacrylate, glycidyl acrylate, glycidyl methacrylate, ethylene glycol diacrylate, ethylene glycol dimethacrylate, polyethylene glycol monoacrylate, and polyethylene glycol monomethacrylate, ethylene type amides such as N-methylol methacrylamide and N-diacetone acrylamide, ethylene type amines such as aminoethyl acrylate, aminoethyl methacrylate, N,N-dimethylaminoethyl acrylate, N,N-dimethylaminoethyl methacrylate, N,N-diethylaminoethyl acrylate, N,N-diethylaminoethyl methacrylate, N,N,N-trimethylaminoethyl acrylate, N,N,N-trimethylaminoethyl methacrylate, as well as the salts thereof.

If the hydrophilic monomers having a carboxyl group in the structure are used for preparing the acrylic type polymer emulsion, a neutralizer can be used to neutralize the carboxyl groups incorporated in the molecule to achieve superior dispersion of the acrylic type polymer into water. Examples of the neutralizer for said carboxyl group include triethylamine, trimethylamine, 2-amino-2-methyl-1-propanol, triethanolamine, potassium hydroxide, and sodium hydroxide.

The monomers containing acrylic type ester monomers for preparing the acrylic type polymer pertaining to the present invention can be used individually or in combinations of two or more kinds; a preferable combination has 70-100 mole% hydrophobic monomers and 0-30 mole% hydrophilic monomers, and a more preferable combination has 85-99 mole% hydrophobic monomers and 1-15 mole% hydrophilic monomers.

For said acrylic type polymer of the present invention, a polymer polymerized from monomers whose main ingredient is an acrylic type ester monomer consisting of an acrylic ester and/or methacrylic ester is preferable. More preferable is a polymer from monomers whose main ingredient is ethyl acrylate and/or ethyl methacrylate, and even more preferable is a polymer from monomers whose main ingredient is ethyl acrylate.

If hydrophilic monomers are to be included, acrylic acid and/or methacrylic acid are preferable for said monomers.

Examples of a specific embodiment of the preferable acrylic type polymer in the present invention include the following formula (1):

[Chemical formula 1-1]

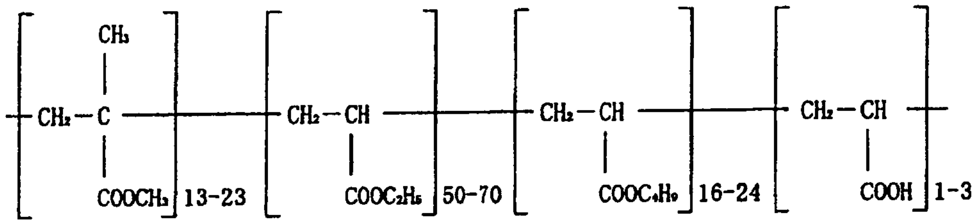

··· (1)

The acrylic type polymer emulsion used in the present invention should preferably contain sulfonated polyvinyl alcohol as an emulsifier/colloid agent to obtain finely textured film. It is particularly preferably to have this for said emulsification polymerization.

Examples of preferably used sulfonated polyvinyl alcohol include the following formula (2) :

[Chemical formula 1-2]

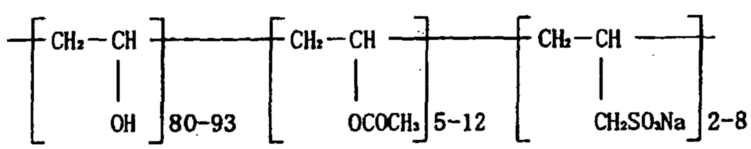

··· (2)

Therefore, the water dispersion of the acrylic type polymer of the present invention should preferably contain sulfonated polyvinyl alcohol.

Also, when preparing said acrylic type polymer emulsion, it is preferable to add a surfactant to stabilize dispersion at the time of the emulsion polymerization. Selection of the surfactant used is not limited in particular; a common anionic, cationic, or non-ionic surfactant can be used. It is also possible to use two or more kinds, such as a combination of an anionic type and non-ionic type and a combination of a cationic type and non-ionic type. Of said surfactants, a non-ionic surfactant is preferable.

Examples of the non-ionic surfactant include polyoxyethylene (hereafter "POE") alkyl ether, POE alkylphenyl ether, and POE-polypropylene oxide (hereafter "POP") block copolymer; examples of the anionic surfactant include alkylbenzene sulfonate, and alkylnaphthalene sulfonate, and polyethylene oxide alkyl ether sulfate. Examples of the cationic surfactant include primary, secondary, and tertiary amine salts, and quaternary ammonium salts having an aliphatic hydrocarbon group. A preferable non-ionic surfactant is POE alkyl ether, of which even more preferable is an alkyl ether having 12-20 POE carbons; POE oleyl ether is particularly preferable. The POE addition mole number is 30-65 moles, and more preferable is 40-60 moles.

The amount of the surfactant added is preferably 5 weight parts or less, more preferably 3 weight parts of less, per 100 weight parts of the monomers used. If it is over 5 weight parts then the physical properties of the film deteriorates.

In the present invention, the following is even more preferable for said acrylic type polymer and the water dispersion of the acrylic polymer (acrylic polymer emulsion or water dispersion thereof). That is:

For the film strength of the acrylic type polymer, the strength is 0.1-100 $kg/cm^2$, more preferably 10-70 $kg/cm^2$. If the strength is less than 0.1 $kg/cm^2$, then the film cannot follow the skin movements and peeling easily occurs. If it is over 100 $kg/cm^2$, then the film causes discomfort on the skin. The elongation is 300-2000%, preferably 500-1000%. If the elongation is less than 300%, then the film formed on the skin cannot follow the skin movements very well. If it is over 2000%, then the wrinkle reduction effect becomes insufficient. The glass transition temperature (Tg) of the acrylic type polymer is preferably 0°C or lower.

The average particle size of the acrylic type polymer in the water dispersion of the acrylic type polymer is 100-600 nm. If the average particle size is less than 100 nm, then there is a sufficient small-wrinkle reduction effect but on the other hand the large-wrinkle reduction effect becomes insufficient. If the average particle size is over 600 nm, then adhesion to the skin becomes poor and peeling tends to occur.

The blend ratio of the acrylic polymer is preferably 1-20 wt% of the total amount of the skin treatment composition. If the blend ratio is less than 1% then the effect of the present invention cannot be obtained sufficiently; if it is over 20 wt% then the viscosity of the skin treatment composition become high and preparation of formulations and application on the skin become difficult. A more preferable blend ratio range is 5-15 wt% of the total amount of the skin treatment composition.

The present invention uses said film forming polymers of the present invention as the main ingredient and preferably consists of these polymers; however, other film forming polymers can be added within the range that does not affect the effect of the present invention, as long as the film shrinkage rate of the total film forming polymer is 20% or less.

The skin treatment composition of the present invention can further contain scaly silica; this reinforce the wrinkle reduction effect of the film consisting of the polyurethane and acrylic type polymer as described above. Specifically, the film formed by the water dispersion of the polymer becomes thick and the wrinkle reduction effect is augmented and reinforced and thus a skin treatment composition for wrinkle reduction that can accommodate the elasticity and movement of the skin for a long duration of time can be obtained.

Scaly silica is scale-like silica particles that have a film forming capability of their own and are able to form a strong film at ordinary temperatures. Scaly silica has a laminated particle configuration; it substantially consists of leaf-like secondary particles formed by multiple thin flake primary particles stacked together with their planes oriented parallel to each other. In the present invention, it is preferable to use micro-scaly silica.

Scaly silica is commercially available; examples of commercial products that can be used include Sunlovely LFS-C (from Dohkai Chemical Industries Co., Ltd.).

In the present invention, a preferable blend ratio of scaly silica is 0.1-5.0 wt%, more preferably 0.5-3.0 wt%, of the total amount of the skin treatment composition. If the blend ratio is less than 0.1 wt%, then the effect of adding scaly silica cannot be manifested sufficiently; if it is over 5.0 wt% then whiteness is seen on the skin surface, which is visually less agreeable.

In addition to the aforementioned ingredients, other ingredients used in skin treatment compositions such as cosmetics and drugs can be blended as necessary into the skin treatment composition of the present invention as long as the effect of the present invention is not adversely affected. The aforementioned optional ingredients include oil components, power ingredients other than those mentioned above, surfactants, humectants, water soluble polymers, thickeners, film forming agents other than those of the present invention, ultraviolet absorbents, sequestering agents, sugars, amino acids, organic amines, pH adjustment agents, nutritional supplements for skin, vitamins, antioxidants, and perfumes.

The skin treatment composition for wrinkle reduction of the present invention can be prepared by blending in the aforementioned ingredients and following a conventional method.

The skin treatment composition of the present invention is used in foundation cosmetic formulations in the form of cream, emulsion, or lotion.

[Invention of claims 13-25]

The best embodiments of the present invention are described in detail below.

The non-emulsifying type cross-linked silicone of the present invention is described in detail below.

Non-emulsifying type cross-linked silicone is a cross-linked silicone in which some of silicone chains are cross-linked, characterized by not having its own ability to emulsify oil and water. A cross-linked silicone is verified as non-emulsifying when a composition having water, oil, and the cross-linked silicone is stirred at a high speed using a homomixer and as a result emulsification does not occur or emulsification occurs but the particle size of the emulsified particles is large, 50 micrometers or more, and the emulsified state does not last when allowed to stand for a while.

Examples of the non-emulsifying cross-linked silicone used in the present invention include a cross polymer derived from a reaction between methyl hydrogen polysiloxane and methylvinyl polysiloxane (hereafter "dimethicone/vinyldimethicone cross polymer"), a cross polymer derived from a reaction between partial long chain alkylated methyl hydrogen polysiloxane and methylvinyl polysiloxane (hereafter "vinyldimethicone/alkyldimethicone cross polymer"), and a cross polymer derived from a reaction between methyl hydrogen polysiloxane and alkene (hereafter "dimethicone cross polymer"). In the present invention, it is preferable to use one, two or more types selected from a group of the aforementioned three types of cross polymers.

For the aforementioned methylvinyl polysiloxane, those that have at least two vinyl groups in the molecule are used in the present invention to effectively produce cross polymers. For this production, methylvinyl polysiloxane having one vinyl group in the molecule is commonly used and this controls the cross-link ratio of the cross-polymer.

The number of carbons in the long chain alkyl in the partial long chain alkylated methyl hydrogen polysiloxane can be set at will; in this invention a preferable number is 10-14 and a lauryl group, having 12 carbons, is the most preferable.

For the aforementioned alkene, those that have at least two vinyl groups in the molecule are used in the present invention to effectively produce cross polymers. For this production, alkene having one vinyl group in the molecule is commonly used and this controls the cross-link ratio of the cross-polymer.

In the present invention, the aforementioned dimethicone/vinyldimethicone cross polymer corresponds to INCI designation "dimethicone/vinyldimethicone cross polymer" or "polysilicone-11". Dimethicone cross polymer corresponds to INCI designation "dimethicone cross polymer". 0f vinyldimethicone/alkyldimethicone cross polymers, a cross polymer derived from a reaction between laurylated methyl hydrogen polysiloxane and methyl vinyl polysiloxane (hereafter "vinylmethicone/lauryldimethicone cross polymer) corresponds to INCI designation "vinyldimethicone/lauryldimethicone cross polymer".

The blend ratio of the non-emulsifying cross-linked silicone is preferably 0.5-5.0 wt% of the total amount of the skin treatment composition. If the blend ratio is less than 0.5 wt% then the effect of the present invention is hard to obtain; on the other hand, adding more than 5.0 wt% would not increase the effect and stickiness would result.

Selection of the liquid oil component used in the present invention is not limited in particular as long as it is a liquid oil component that can be blended into a liquid cosmetic at an ordinary temperature (25˚C). Examples of the liquid oil component include liquid silicone oil, liquid hydrocarbon oil, liquid ester oil, and liquid higher aliphatic acid.

Examples of said liquid silicone oil include straight chain or cyclic silicone oil; specific examples include dimethyl silicone, decamethylcyclopentasiloxane, octamethylcyclotetrasiloxane, phenyl dimethicone, and octyl trimethicone.

Examples of said liquid hydrocarbon oil include liquid petrolatum, squalane, light isoparaffin, $\alpha$-olefin oligomers, and isodecane.

Examples of said liquid ester oil include glyceryl tri-2-ethylhexanoate, diisobutyl adipate, di-2-ethylhexyl succinate, cetyl 2-ethylhexanoate, 2-hexyldecyl 2-ethylhexanoate, neopentyl glycol di-2-ethylhexanoate, glyceryl tri-2-ethylhexanoate, trimethylolpropane tri-2-ethylhexanoate, glyceryl tri- (caprylate/caprate), neopentyl glycol dicaprate, 2-ethylhexyl isononanoate, isononyl isononanoate, isodecyl isononanoate, isotridecyl isononanoate, 2-octyldecyl myristate, isopropyl palmitate, 2-ethylhexyl palmitate, 2-hexyldecyl stearate, cetyl isostearate, isopropyl isostearate, 2-hexyldecyl isostearate, isostearyl isostearate, isodecyl pivalate, isostearyl pivalate, 2-octyldodecyl pivalate, 2-octyldodecyl dimethyloctanoate, 2-ethylhexyl hydroxystearate, 2-octyldodecyl 12-stearoylstearate, oleyl oleate, 2-ethylhexyl salicylate, and jojoba oil.

Examples of said liquid higher aliphatic acid include isostearic acid.

The liquid oil component in the present invention is blended in as a constituent ingredient of the skin treatment composition of the present invention; it also functions as a swelling agent of said non-emulsifying cross-linked silicone.

In the present invention, when preparing the skin treatment composition, it is preferable to blend in the non-emulsifying cross-linked silicone in a swollen form (gel-like composition) swollen with said liquid oil component. This way, a skin treatment composition having a superior effect can be prepared in a stable form.

For the liquid oil component for this purpose, a liquid oil component having a low viscosity at ordinary temperatures, 100 mPa·s or less for example, is preferable. A preferable viscosity range is 1-100 mPa·s.

In the non-emulsifying cross-linked silicone swollen with the liquid oil, which is a preferable form for blending into the present invention, a preferable mass ratio of the non-emulsifying cross-linked silicone and the liquid oil is 5-40: 95-60. Within this mass ratio range it is a preferable swollen material for the skin treatment composition of the present invention.

Said swollen form of the non-emulsifying cross-linked silicone is commercially available and therefore such commercial products can be used, examples of which follow.

Examples of a swollen form of INCI designation dimethicone/vinyldimethicone cross polymer or polysilicone-11 include KSG-15 ((a mixture of dimethicone/vinyldimethicone) cross polymer and cyclopentasiloxane, approximately 5% of which is cross-linked), KSG-16 ((a mixture of dimethicone/vinyldimethicone) cross polymer and dimethicone 6 mPa·s, approximately 25% of which is cross-linked), KSG-18 ((a mixture of dimethicone/vinyldimethicone) cross polymer and phenyltrimethicone, approximately 15% of which is cross-linked) (these are from Shin-Etsu Chemical Co., Ltd.), GRANSIL GCM (a mixture of polysilicone-11 and octamethylcyclotetrasiloxane, approximately 6% of which is cross-linked), GRANSIL GCM-5 (a mixture of polysilicone-11 and decamethylcyclopentasiloxane, approximately 6% of which is cross-linked), GRANSIL IDS (a mixture of polysilicone-11 and isodecane, approximately 7% of which is cross-linked), GRANSIL DMG-6 (a mixture of polysilicone-11 and dimethicone 6 mPa·s, approximately 18% of which is cross-linked), GRANSIL DMG-20 (a mixture of polysilicone-11 and dimethicone 20 mPa·s, approximately 25% of which is cross-linked), GRANSIL DMG-50 (a mixture of polysilicone-11 and dimethicone 50 mPa·s, approximately 26% of which is cross-linked), and GRANSIL PM (a mixture of polysilicone-11 and phenyltrimethicone, approximately 20% of which is cross-linked), and GRANSIL ININ (a mixture of polysilicone-11 and isononyl isononanoate, approximately 15% of which is cross-linked) (these are from GRANT Inc.).

Examples of a swollen form of INCI designation vinyldimethicone/lauryldimethicone cross polymer include KSG-41 ((a mixture of vinyldimethicone/lauryldimethicone) cross polymer and liquid petrolatum, approximately 30% of which is cross-linked), KSG-42 ((a mixture of vinyldimethicone/lauryldimethicone) cross polymer and light isoparaffin,

approximately 25% of which is cross-linked), KSG-43 ((a mixture of vinyldimethicone/lauryldimethicone) cross polymer and glyceryl tri-2-ethylhexanoate, approximately 30% of which is cross-linked), and KSG-44 ((a mixture of vinyldimethicone/lauryldimethicone) cross polymer and squalane, approximately 5% of which is cross-linked), all of which are from Shin-Etsu Chemical Co., Ltd.

Examples of a swollen form of INCI designation dimethicone cross polymer include DC9040 (a mixture of dimethicone cross polymer and decamethylcyclopentasiloxane, approximately 12% of which is cross-linked), DC9041 (a mixture of dimethicone cross polymer and dimethicone 5 mPa·s, approximately 16% of which is cross-linked), and DC9045 (a mixture of dimethicone cross polymer and decamethylcyclopentasiloxane, approximately 12.5% of which is cross-linked), all of which are from Dow Corning Toray.

The blend ratio of the liquid oil component is preferably 0.5-30 wt% of the total amount of the skin treatment composition. Within this blend ratio range, the effect of the present invention can be sufficiently obtained.

The skin treatment composition for wrinkle reduction of the present invention further contains as an essential ingredient a water insoluble film forming polymer having a film shrinkage rate of 20% or less; the main ingredient of said film forming polymer is polyurethane having a film shrinkage rate of 20% or less. The film shrinkage rate is defined as described above.

In the present invention, the film forming polymer preferably consists of polyurethane having a film shrinkage rate of 20% or less. However, the present invention does not reject the addition of other film forming polymers within the range that does not adversely affect the effect of the present invention, provided that the film shrinkage rate of the total film forming polymer is 20% or less.

Those for which said film shrinkage rate is over 20% have a poor wrinkle reduction effect, peel off the skin, cause noticeable "gloss", and exhibit poor usability due to discomfort; therefore they cannot manifest the effects of the present invention. The preferable range of the film shrinkage rate is 10% or less. The lower limit of the film shrinkage rate is 0, i.e. no shrinkage, which is preferable; however, 5% is sufficient and also industrially practical. That is, the film shrinkage rate in the present invention is 0-20%; within this range, preferable combinations of the upper and lower limits are used, examples include 5-20%, 0-10%, and 5-10%.

The polyurethane used in the present invention is a polymer having urethane bonds; the urethane bond is formed by an addition reaction between an isocyanate group and a compound having active hydrogen, such as a hydroxyl group. The polyurethane in the present invention is preferably obtained with a conventional method reacting at least (A) an isocyanate compound having two isocyanate groups and (B) a diol compound having two hydroxyl groups.

Selection of said isocyanate compound (ingredient A) is not limited in particular as long as it is used in conventional polyurethane manufacturing, examples for which include organic diisocyanate compounds such as aliphatic diisocyanate compounds, alicyclic isocyanate compounds, and aromatic diisocyanate compounds. More preferable are aliphatic diisocyanate compounds and alicyclic diisocyanate compounds. One, two or more isocyanate compounds are freely selected and used.

Examples of said aliphatic diisocyanate compounds include ethylene diisocyanate, 2,2,4-trimethylhexamethylene diisocyanate, and 1,6-hexamethylene diisocyanate.

Examples of said alicyclic diisocyanate compounds include hydrogenated 4,4'-diphenylmethane diisocyanate, 1,4-cyclohexane diisocyanate, methylcyclohexylene diisocyanate, isophorone diisocyanate (hereafter referred to as "IPDI"), and norbornane diisocyanate.

Examples of said aromatic diisocyanate compound include 4,4'-diphenylmethane diisocyanate, xylylene diisocyanate, toluene diisocyanate, and naphthalene diisocyanate.

Of the specific examples of said isocyanate compound (ingredient A), 1,6-hexymethylene diisocyanate, IPDI, and norbornane diisocyanate are preferable due to superior weather resistance and availability. IPDI is particularly preferable.

Selection of said diol compound (ingredient B) is not limited in particular as long as it is used in conventional polyurethane manufacturing, preferable examples for which include an alkylene diol, carboxyl group-containing alkyl diol, alicyclic diol, spiro diol, polyester diol, polyether diol, polycarbonate diol, polybutadiene diol, polyisoprene diol, and polyolefin diol. Of these, particularly preferably used are alkylene diol, carboxyl group-containing alkylene diol, alicyclic diol, polyether diol, and polycarbonate diol. One, two or more diol compounds are freely selected and used.

Examples of said alkylene diol include ethylene glycol, propylene glycol, 1,4-butane diol, 1,6-hexane diol, neopentyl glycol, 1,8-octane diol, and 1,10-decane diol.

Preferable examples of said carboxyl group-containing alkylene diol include carboxylic acid having 3-26 carbons, more preferably 3-12 carbons, as well as having a dialylol groups, such as dimethylol, diethanol and propanol. Specific examples include dimethylol propionic acid (hereafter "DMPA") and dimethylol butanoic acid (hereafter "DMBA"); a mixture thereof can be used as well.

In the present invention, when a carboxyl group-containing alkylene diol is used for the diol compound in the synthesis to obtain polyurethane containing carboxyl groups in the molecule, the carboxyl groups incorporated in the molecule can be neutralized by neutralizers such as triethylamine, trimethylamine, 2-amino-2-methyl-1-propanol, triethanolamine, potassium hydroxide, and sodium hydroxide to obtain a stable water dispersion containing polyurethane, which is a preferred embodiment described later.

Examples of said alicyclic diol include 1,4-cyclohexane dimethanol (hereafter "CHDM"), which is preferable because it gives adequate strength to the film.

Preferable examples of said spiro diol include spiro glycol.

Examples of said polyester diol include those obtained by condensation polymerization between al least one chosen from a group of dicarboxylic acids including succinic acid, glutaric acid, adipic acid, sebacic acid, azelaic acid, maleic acid, fumaric acid, phthalic acid, and terephthalic acid and at least one chosen from a group of diols including ethylene glycol, propylene glycol, 1,4-butane diol, 1,6-hexane diol, neopentyl glycol, 1,8-octane diol, 1,10-decane diol, diethylene glycol, polyethylene glycol (hereafter "PEG"), polypropylene glycol (hereafter "PPG"), tetramethylene glycol, polytetramethylene glycol, and spiro glycol, as well as those obtained by the ring opening polymerization of lactonic acid.

Examples of said polyether diol include polyether diols contained in diols used in the synthesis of said polyester diol, examples of which include polyoxyethylene diols such as diethylene glycol and PEG, polyoxypropylene diols such as PPG, polyoxytetramethylene diols such as polytetramethylene glycol (hereafter "PTMG"), phenols such as bisphenol A, and a product of the ring opening addition polymerization of bisphenol A and at least one compound chosen from propylene oxide (hereafter "P0") and ethylene oxide (hereafter "E0") (if a copolymer is used, such a copolymer can be either a block copolymer or random copolymer). 0f these, PEG, PPG, and PTMG are preferable, and PTMG is particularly preferable since it forms a soft film and adheres to the skin very well.

Preferable examples of said polycarbonate diol include polyhexamethylene carbonate diol (hereafter "PHMC") because of its ability to form a soft film and its superior adhesion to the skin.

In the present invention, polyurethane that forms a film that manifests a superior wrinkle reduction effect can be obtained by using the diol compound (ingredient B) consisting of a mixture of a polyether diol, polycarbonate diol, and carboxyl group-containing alkylene diol.

In the present invention, when mixing a polyether diol, polycarbonate diol, and carboxyl group-containing alkylene diol, it is particularly preferable to use PTMG for said polyether diol, PHMC for the polycarbonate diol, and DMPA and/or DMBA for said carboxyl group-containing alkylene diol.

In the present invention, when using the aforementioned preferable diol compounds, it is particularly preferable to use an isocyanate compound that contains IPDI; a skin treatment composition for wrinkle reduction having the most exceptional wrinkle reduction effect can be obtained by using polyurethane synthesized by using IPDI for the isocyanate and PTMG, PHMC, DMPA and/or DMBA for the diol compounds.

The molar ratio of said isocyanate compound (ingredient A) and diol compound (ingredient B) is preferably A/B = 2/0.8-2/1.8, and more preferably A/B = 2/1-2/1.8.

In the present invention, it is preferable to use polyurethane having structural units derived from alkylene oxide (hereafter "RO"); this makes it easier to control the elongation of the film obtained from the water dispersion of the polyurethane that is a preferred embodiment described later to obtain a flexible film. A skin treatment composition that manifests superior effects and superior usability can be obtained by using this polyurethane for the preparation.

Examples of the compound having a structural unit derived from R0 include a polyoxyethylene diol such as diethylene glycol and PEG, a polyoxypropylene diol such as PPG, a polyoxytetramethylene diol such as PTMG, polyoxyethylene polyoxypropylene glycol (E0/P0 block copolymer), phenols such as bisphenol A, and a product of the ring opening addition polymerization of bisphenol A and at least one compound chosen from P0 and E0 (if a copolymer is used, such a copolymer can be either a block copolymer or random copolymer); preferably used are PEG, PPG, PTMG, etc.

In the present invention, the compound having a structural unit derived from R0 is used as a polyether diol ingredient in said ingredient B.

is preferably blended in as a water dispersion, i.e. dispersed in water, which is an essential ingredient of the present invention. Said water dispersion, when applied on the skin and dried, forms a polymer film on the skin.

In the present invention, when preparing the skin treatment composition, the film forming polymer

The water dispersion of polyurethane is prepared with a conventional method; for example, a pre-polymer having remaining isocyanate obtained from a reaction in an organic solvent is dispersed in water containing potassium hydroxide under high speed agitation, followed by a chain elongation reaction to increase the molecular weight, and said organic solvent is recovered from the obtained water based solution to obtain the water dispersion of the polyurethane.

In the present invention, the following is particularly preferable for said polyurethane and water dispersion of polyurethane. That is:

With regard to the film characteristics of the polyurethane, the strength is 300-700 kg/$cm^2$, and preferably 400-600 kg/$cm^2$. If the strength is less than 300 kg/$cm^2$, then the film cannot follow the skin movements and peeling easily occurs. If it is over 700 kg/$cm^2$, then the film causes discomfort on the skin. The elongation is 200-500%, preferably 300-500%. If the elongation is less than 200%, then the film formed on the skin cannot follow the skin movements very

well. If it is over 500%, then the wrinkle reduction effect becomes insufficient.

The average particle size of the polyurethane in the water dispersion of the polyurethane is 10-300 nm, preferably 20-200 nm. If the average particle size is less than 10 nm, then there is a sufficient small-wrinkle reduction effect but on the other hand the large-wrinkle reduction effect becomes insufficient. If the average particle size is over 300 nm, then adhesion to the skin becomes poor and peeling tends to occur.

A preferable embodiment in the present invention uses two kinds of water dispersions of polyurethane having different particle sizes. This way, not only the wrinkle reduction effect but also a skin mark erasing effect can be obtained. For said two kinds of polyurethane having different particle sizes, polyurethanes having an average particle size of 20-60 nm and an average particle size of 150-200 nm are preferable

Preparation examples of the polyurethane having a film shrinkage rate of 20% or less used in the present invention are described below.

(Preparation example 2-1)

50 g of IPDI, 120 g of PTMG (molecular weight 1,000), 5 g of CHDM, and 10 g of DMBA were put into a four-neck flask equipped with a stirrer, a thermometer, a nitrogen introduction tube, and a reflux cooling apparatus, to which 50 g of ethyl acetate was added as a solvent, and then an oil bath was used to raise the temperature to 80˚C and allow the reaction to proceed for 6 hours to obtain a pre-polymer having remaining isocyanate groups. This pre-polymer having remaining isocyanate groups was cooled down to 50˚C and dispersed in 800 g of water containing 6 g of potassium hydroxide under high speed stirring, followed by 3 hours of a chain elongation reaction at 50˚C to increase the molecular weight. Said ethyl acetate was recovered from the obtained water-based liquid to obtain a water dispersion of polyurethane substantially having no solvent (polyurethane solid content 20 wt%). (Average particle size: 170 nm, film strength: 410 $kg/cm^2$, film elongation: 320%, film shrinkage rate: 11%)

(Preparation example 2-2)

50 g of IPDI, 60 g of PTMG (molecular weight 1,000), 40 g of PHMC (molecular weight 2,000), and 10 g of DMBA were put into a four-neck flask equipped with a stirrer, a thermometer, a nitrogen introduction tube, and a reflux cooling apparatus, to which 50 g of ethyl acetate was added as a solvent, and then an oil bath was used to raise the temperature to 80˚C and allow the reaction to proceed for 3 hours. 2 g of N-methyldiethanolamine (NMDE tA) and 40 g of ethyl acetate were added, followed by 3 hours of reaction at 80˚C to obtain a pre-polymer having remaining isocyanate groups. This pre-polymer having remaining isocyanate groups was cooled down to 50˚C and dispersed in 700 g of water containing 6 g of potassium hydroxide under high speed stirring, followed by 3 hours of a chain elongation reaction at 50˚C to increase the molecular weight. Said ethyl acetate was recovered from the obtained water-based liquid to obtain a water dispersion of polyurethane substantially having no solvent (polyurethane solid content 20 wt%).
(Average particle size: 40 nm, film strength: 530 $kg/cm^2$, film elongation: 360%, film shrinkage rate: 9%)

The blend ratio of the polyurethane having a film shrinkage rate of 20% or less in the present invention is preferably 0.1-10.0 wt% of the total amount of the skin treatment composition. If the blend ratio is less than 0.1 wt% then the effect of the present invention is hard to be obtained sufficiently; and if it is over 10.0 wt% then the film tends to peel off the skin. A more preferable blend ratio range is 1.0-8.0 wt% of the total amount of the skin treatment composition.

As mentioned before, the skin treatment composition of the present invention can contain, in addition to the polyurethane having a film shrinkage rate of 20% or less, other film forming polymers, examples of which include acrylic type polymers. Even when film forming polymers other than the polyurethane having a film shrinkage rate of 20% or less are added, the blend ratio of the total film forming polymers is still preferably in the range of 0.1-10.0 wt%.

The other essential ingredient of the present invention is water. Water other than the water contained in said essential ingredients is further added as appropriate to form the skin treatment composition.

In addition to the aforementioned ingredients, other ingredients used in skin treatment compositions such as cosmetics and drugs can be blended as necessary into the skin treatment composition of the present invention as long as the effect of the present invention is not adversely affected. The aforementioned optional ingredients include, albeit partially redundant, oil components, power ingredients, surfactants, humectants, water soluble polymers, thickeners, ultraviolet absorbents, sequestering agents, sugars, amino acids, organic amines, pH adjustment agents, nutritional supplements for skin, vitamins antioxidants, and perfumes that are not those mentioned above.

The skin treatment composition for wrinkle reduction of the present invention can be prepared by blending in the aforementioned ingredients and following a conventional method.

The skin treatment composition of the present invention is used in foundation cosmetic formulations in the form of a cream, emulsion, lotion, or gel.

EXAMPLES

[Invention of claims 1-12]

(Preparation example 1-1)

Synthesis of a water dispersion of polyurethane

50 g of IPDI, 120 g of PTMG (molecular weight 1,000), 5 g of CHDM, and 10 g of DMBA were put into a four-neck flask equipped with a stirrer, a thermometer, a nitrogen introduction tube, and a reflux cooling apparatus, to which 50 g of ethyl acetate was added as a solvent, and then an oil bath was used to raise the temperature to 80˚C and allow the reaction to proceed for 6 hours to obtain a pre-polymer having remaining isocyanate groups. This pre-polymer having remaining isocyanate groups was cooled down to 50˚C and dispersed in 800 g of water containing 6 g of potassium hydroxide under high speed stirring, followed by 3 hours of a chain elongation reaction at 50˚C to increase the molecular weight. Said ethyl acetate was recovered from the obtained water-based liquid to obtain a water dispersion of polyurethane substantially having no solvent (polyurethane solid content 20 wt%).
(Average particle size: 170 nm, film strength: 410 $kg/cm^2$, film elongation: 320%, film shrinkage rate : 11%)

(Preparation example 1-2)

Synthesis of a water dispersion of polyurethane

50 g of IPDI, 60 g of PTMG (molecular weight 1,000), 40 g of PHMC (molecular weight 2,000), and 10 g of DMBA were put into a four-neck flask equipped with a stirrer, a thermometer, a nitrogen introduction tube, and a reflux cooling apparatus, to which 50 g of ethyl acetate was added as a solvent, and then an oil bath was used to raise the temperature to 80˚C and allow the reaction to proceed for 3 hours. 2 g of N-methyldiethanolamine (NMDE tA) and 40 g of ethyl acetate were added, followed by 3 hours of reaction at 80˚C to obtain a pre-polymer having remaining isocyanate groups. This pre-polymer having remaining isocyanate groups was cooled down to 50˚C and dispersed in 700 g of water containing 6 g of potassium hydroxide under high speed stirring, followed by 3 hours of a chain elongation reaction at 50˚C to increase the molecular weight. Said ethyl acetate was recovered from the obtained water-based liquid to obtain a water dispersion of polyurethane substantially having no solvent (polyurethane solid content 20 wt%).
(Average particle size: 40 nm, film strength: 530 $kg/cm^2$, film elongation: 360%, film shrinkage rate : 9%)

(Preparation example 1-3)

Synthesis of a water dispersion of an acrylic type polymer (acrylic type polymer emulsion) 100 g of ion-exchanged water and 2 g of polyoxyethylenecetyl ether were put into a four-neck flask equipped with stirring blades, a thermometer, a nitrogen introduction tube, and a reflux cooling apparatus; the mixture was heated and stirred as nitrogen gas was instilled into it and the liquid temperature was maintained at 80˚C. Meanwhile, a monomer mixed solution consisting of 40 g of ion-exchanged water, 1 g of sodium laurylsulfate, 1 g of polyoxyethylenecetyl ether, 50 g of methyl methacrylate, 42 g of 2-ethylhexyl acrylate, 3 g of methacrylic acid, and 1.4 g of sulfonated polyvinyl alcohol and an initiator aqueous solution containing 0.3 parts potassium persulfate and 10 parts ion-exchanged water were prepared. 5 wt% of the monomer mixed solution and 10 wt% of the initiator aqueous solution were added to a four-neck flask and stirred to initiate an emulsification polymerization reaction; the rest of the monomer mixed solution and the initiator aqueous solution were concurrently dripped into the four-neck flask over a period of approximately three hours. Stirring was continued for another hour as the liquid temperature was kept at 80˚C and then the obtained reaction mixture was cooled down to 50˚C. Aqueous ammonia was then added to adjust the pH to approximately 8 and the temperature was cooled down to room temperature to obtain the target water dispersion of acrylic type polymer (acrylic type polymer solid content 50 wt%). (Average particle size: 480 nm, film strength: 20 $kg/cm^2$, film elongation: 700%, film shrinkage rate : 6%)

(Preparation example 1-4)

Synthesis of a water dispersion of an acrylic type polymer (acrylic type polymer emulsion) 100 g of ion-exchanged water and 2 g of polyoxyethylenestearyl ether were put into a four-neck flask equipped with stirring blades, a thermometer, a nitrogen introduction tube, and a reflux cooling apparatus; the mixture was heated and stirred as nitrogen gas was instilled into it and the liquid temperature was maintained at 80˚C. Meanwhile, a monomer mixed solution consisting of 40 g of ion-exchanged water, 1 g of sodium polyoxyethylene laurylsulfate, 1 g of polyoxyethylene stearyl ether, 30 g of methyl methacrylate, 67 g of 2-ethylhexyl acrylate, and 3 g of methacrylic acid and an aqueous solution containing 0.3

parts potassium persulfate and 10 parts ion-exchanged water were prepared. 5 wt% of the monomer mixed solution and 10 wt% of the initiator aqueous solution were added to a four-neck flask and stirred to initiate an emulsification polymerization reaction; the rest of the monomer mixed solution and the initiator aqueous solution were concurrently dripped into the four-neck flask over a period of approximately three hours. Stirring was continued for another hour as the liquid temperature was kept at 80˚C and then the obtained reaction mixture was cooled down to 50˚C. Aqueous ammonia was then added to adjust the pH to approximately 8 and the temperature was cooled down to room temperature to obtain the target water dispersion of an acrylic type polymer (acrylic type polymer solid content 50 wt%).
(Average particle size: 100 nm, film strength: 50 kg/cm$^2$, film elongation: 500%, film shrinkage rate: 7%)

(Method for evaluating/measuring the film)

1. Film shrinkage rate

A water dispersion of a solid equivalent 1 g of the polymer (polyurethane, acrylic type polymer, etc.) was poured into a 5 cm x 5 cm polyethylene mold such that the film thickness is approximately 0.5 mm and dried at a prescribed temperature (50˚C) for a prescribed amount of time (three days at room temperature) to obtain a film, and vertical height and horizontal length of this film were measured and used in the following formula to calculate the film shrinkage rate.
$$\text{Film shrinkage rate (\%)} = [(\text{Vertical measurement} \times \text{Horizontal measurement})/25] \times 100$$

2. Measurement of the strength and elongation of the film

A water dispersion of a solid equivalent 1 g of the polymer (polyurethane, acrylic type polymer, etc.) was poured into a 5 cm x 5 cm polyethylene mold such that the film thickness is approximately 0.5 mm and dried at a prescribed temperature (50˚C ) for a prescribed amount of time (three days at room temperature) to obtain a film, and this film was cut out using a dumbbell #3 for the measurement. The autograph function of "Tensile tester RTM-250" from Orientec Co., Ltd. was used to carry out the measurement of the strength and elongation at 20˚C and a cross head speed of 300 mm/min.

3. Particle size measurement of the water dispersion

The water dispersion of the polymer (polyurethane, acrylic type polymer, etc.) was measured with a laser light scattering particle size distribution measuring apparatus from 0tsuka Electronics Co., Ltd.
The present invention is described in detail below by referring to Examples. The blend ratios are in wt% units. Before the description of Examples, the efficacy test method used in the present invention is described.

(Sensory test)

Each specimen was tested by a panel of 10 specialists for the sensation during use. Each specimen was evaluated based on the following criteria for usability and the sensation during use, including (1) the wrinkle reduction effect right after application, (2) the wrinkle reduction effect five hours after application, (3) peeling off the skin (right after application, three hours after application), (4) glossiness (skin greasy and shining), (5) no stickiness (right after application, three hours after application), and (6) no discomfort.

"Evaluation criteria"

(1) Wrinkle reduction effect right after application

◎ : 8 or more felt a wrinkle reduction effect.
○ : 5-7 felt a wrinkle reduction effect.
△ : 3-4 felt a wrinkle reduction effect.

× : 2 or fewer felt a wrinkle reduction effect.

(2) Wrinkle reduction effect five hours after application

◎ : 8 or more felt a wrinkle reduction effect.
○ : 5-7 felt a wrinkle reduction effect.
△ : 3-4 felt a wrinkle reduction effect.
× : 2 or fewer felt a wrinkle reduction effect.

(3) Peeling off from the skin

◎ : 8 or more felt there was no peeling off.
○ : 5-7 felt there was no peeling off.
△ : 3-4 felt there was no peeling off.
× : 2 or fewer felt there was no peeling off.

(4) Glossiness

◎ : 8 or more felt there was no glossiness.
○ : 5-7 felt there was no glossiness.
△ : 3-4 felt there was no glossiness.
× : 2 or fewer felt there was no glossiness.

(5) No stickiness

◎ : 8 or more felt there was no stickiness.
○ : 5-7 felt there was no stickiness.
△ : 3-4 felt there was no stickiness.
× : 2 or fewer felt there was no stickiness.

(6) No discomfort

◎ : 8 or more felt there was no discomfort.
○ : 5-7 felt there was no discomfort.
△ : 3-4 felt there was no discomfort.
× : 2 or fewer felt there was no discomfort.

"Examples 1-1 to 1-13, Comparative examples 1-1 to 1-9"

Skin treatment compositions for wrinkle reduction were prepared by mixing ingredients with blend ratios (total blend ratio 100 wt%) according to the recipes shown in Tables 1-1 to 1-3. Also, the efficacy test was conducted on the skin treatment compositions for wrinkle reduction from the aforementioned Examples 1-1 to 1-13 and Comparative examples 1-1 to 1-9; the evaluation results are also shown in Tables 1-1 to 1-3.
[Table 1-1]

| | Example | | | | | | | | |
|---|---|---|---|---|---|---|---|---|---|
| | 1-1 | 1-2 | 1-3 | 1-4 | 1-5 | 1-6 | 1-7 | 1-8 | 1-9 |
| Ion-exchanged water | Balance | Balance | Balance | Balance | Balance | Balance | Balance | Balance | Balance |
| Glycerin | 6.0 | 6.0 | 6.0 | 6.0 | 6.0 | 6.0 | 6.0 | 6.0 | 6.0 |
| Water dispersion of polyurethane (solid content 20 wt%) (preparation example 1) | 10.0 | 5.0 | 10.0 | - | - | 5.0 | 45.0 | 10.0 | 10.0 |
| Water dispersion of polyurethane (solid content 20 wt%) (preparation example 2) | - | 5.0 | - | 10.0 | 10.0 | - | - | 10.0 | 20.0 |
| Water dispersion of an acrylic type polymer (solid content 50 wt%) (preparation example 3) | 10.0 | 10.0 | - | 10.0 | - | 5.0 | - | 15.0 | 10.0 |
| Water dispersion of an acrylic type polymer (solid content 50 wt%) (preparation example 4) | - | - | 10.0 | - | 10.0 | - | 30.0 | - | 10.0 |
| Total | 100 | 100 | 100 | 100 | 100 | 100 | 100 | 100 | 100 |
| Wrinkle reduction effect right after application | ◎ | ◎ | ◎ | ◎ | ◎ | ◎ | ◎ | ◎ | ◎ |
| Wrinkle reduction effect five hours after application | ◎ | ◎ | ◎ | ◎ | ◎ | ○ | ◎ | ◎ | ◎ |
| Peeling | ◎ | ◎ | ◎ | ◎ | ◎ | ◎ | ○ | ◎ | ◎ |
| Glossiness | ◎ | ◎ | ◎ | ◎ | ◎ | ◎ | ○ | ◎ | ◎ |
| No stickiness | ◎ | ◎ | ◎ | ◎ | ◎ | ◎ | ○ | ◎ | ◎ |
| No discomfort | ◎ | ◎ | ◎ | ◎ | ◎ | ◎ | ◎ | ◎ | ◎ |

[Table 1-2]

| | Example | | | |
|---|---|---|---|---|
| | 1-10 | 1-11 | 1-12 | 1-13 |
| Ion-exchanged water | Balance | Balance | Balance | Balance |
| Glycerin | 6.0 | 6.0 | 6.0 | 6.0 |
| Water dispersion of polyurethane (solid content 20 wt%) (preparation example 1) | 5.0 | 5.0 | 5.0 | 5.0 |
| Water dispersion of polyurethane (solid content 20 wt%) (preparation example 2) | 5.0 | 5.0 | 5.0 | 5.0 |
| Water dispersion of an acrylic type polymer (solid content 50 wt%) (preparation example 3) | 10.0 | 5.0 | 10.0 | 5.0 |
| Water dispersion of an acrylic type polymer (solid content 50 wt%) (preparation example 4) | - | 5.0 | - | 5.0 |
| Water dispersion of scaly silica (solid content 15 wt%) (Note 6) | 10.0 | 10.0 | 4.0 | 20.0 |
| Total | 100 | 100 | 100 | 100 |
| Wrinkle reduction effect right after application | ◎ | ◎ | ◎ | ◎ |
| Wrinkle reduction effect five hours after application | ◎ | ◎ | ◎ | ◎ |
| Peeling | ◎ | ◎ | ◎ | ◎ |
| Glossiness | ◎ | ◎ | ◎ | ◎ |
| No stickiness | ◎ | ◎ | ◎ | ◎ |
| No discomfort | ◎ | ◎ | ◎ | ◎ |

[Table 1-3]

| | Comparative example | | | | | | | | |
|---|---|---|---|---|---|---|---|---|---|
| | 1-1 | 1-2 | 1-3 | 1-4 | 1-5 | 1-6 | 1-7 | 1-8 | 1-9 |
| Ion-exchanged water | Balance | Balance | Balance | Balance | Balance | Balance | Balance | Balance | Balance |
| Glycerin | 6.0 | 6.0 | 6.0 | 6.0 | 6.0 | 6.0 | 6.0 | 6.0 | 6.0 |
| Water dispersion of polyurethane (solid content 20 wt%) (preparation example 1) | - | - | - | 10.0 | 10.0 | - | - | - | - |
| Water dispersion of an acrylic type polymer (solid content 50 wt%) (preparation example 3) | - | - | - | - | - | - | - | 10.0 | 10.0 |
| Commercially available water dispersion of polyurethane (solid content 33 wt%) (Note 1) | 10.0 | - | - | - | - | - | - | - | - |
| Commercially available water dispersion of polyurethane (solid content 39 wt%) (Note 2) | - | 10.0 | - | - | - | - | - | - | - |
| Commercially available water dispersion of polyurethane (solid content 20 wt%) (Note 3) | - | - | 10.0 | - | - | - | - | - | 10.0 |
| Commercially available water dispersion of an acrylic type polymer (solid content 50 wt%) (Note 4) | - | - | - | - | 10.0 | 10.0 | - | - | - |
| Commercially available water dispersion of an acrylic type polymer (solid content 48 wt%) (Note 5) | - | - | - | - | - | - | 10.0 | - | - |
| Total | 100 | 100 | 100 | 100 | 100 | 100 | 100 | 100 | 100 |
| Wrinkle reduction effect right after application | ○ | ○ | ○ | △ | ○ | ○ | ○ | △ | ○ |
| Wrinkle reduction effect five hours after application | × | × | × | × | × | × | × | × | × |
| Peeling | × | × | × | △ | × | × | × | △ | × |
| Glossiness | × | × | × | △ | × | × | × | △ | × |
| No stickiness | ○ | ○ | ○ | ○ | × | × | × | × | × |
| No discomfort | × | × | × | ○ | × | × | × | ○ | × |

In Tables 1-1 to 1-3:

(Note 1) Avalure UR405 (from NOVEON)
(Average particle size: 100 nm, strength: 40 kg/cm$^2$, elongation: 150%, film shrinkage rate: 35%)
(Note 2) Avalure UR445 (from NOVEON)
(Average particle size: 5 nm, strength: 240 kg/cm$^2$, elongation: 610%, film shrinkage rate: 27%)
(Note 3) Iodosol PUD (from Nippon NSC)

(Average particle size: 20 nm, strength: 200 $kg/cm^2$, elongation: 530%, film shrinkage rate: 23%)
(Note 4) Dashcoat (from Daito Kasei Kogyo Co., Ltd.)
(Average particle size: 150 nm, strength: 15 $kg/cm^2$, elongation: 700%, film shrinkage rate: 25%)
(Note 5) Ultrasol 2075C (from Ganz Chemical Co., Ltd.)
(Average particle size: 200 nm, strength: 40 $kg/cm^2$, elongation: 1000%, film shrinkage rate: 13%)
(Note 6) Sunlovely LFS-C (solid content 15%) (from Dohkai Chemical Industries Co., Ltd.)

As clearly shown in Tables 1-1 to 1-3, the skin treatment compositions for wrinkle reduction from Examples 1-13 pertaining to the present invention are superior in terms of the wrinkle reduction effect and free of peeling, glossiness, stickiness, and discomfort. On the contrary, skin treatment compositions for wrinkle reduction from Comparative examples 1-9, which do not meet the constituent requirements of the present invention, do not manifest the effects of the present invention.

More Examples of the skin treatment composition for wrinkle reduction of the present invention are described below. The aforementioned efficacy test was conducted on these and the results were superior for all of these.

[Example 1-14] Skin treatment composition for wrinkle reduction (cream type)

| | Ingredients | Blend ratio (wt%) |
|---|---|---|
| (1) | Stearyl alcohol | 6.0 |
| (2) | Stearic acid | 2.0 |
| (3) | Hydrated lanolin | 4.0 |
| (4) | Squalane | 9.0 |
| (5) | 5-0ctyldodecanol | 10.0 |
| (6) | 1,3-butylene glycol | 6.0 |
| (7) | Water dispersion of polyurethane from preparation example 1 | 5.0 |
| (8) | Water dispersion of polyurethane from preparation example 2 | 10.0 |
| (9) | Water dispersion of an acrylic type from preparation example 3 | polymer 8.0 |
| (10) | PEG 1500 | 4.0 |
| (11) | Polyoxyethylene (25) cetyl alcohol ether | 3. 0 |
| (12) | Glycerin monostearate | 2.0 |
| (13) | Ethylparaben | 0. 1 |
| (14) | Butylparaben | 0. 1 |
| (15) | Tocopherol | 0.01 |
| (16) | Perfume | 0. 1 |
| (17) | Purified water | Balance |
| (18) | Scaly silica slurry (Note 1) | 5.0 |
| | | Total 100.0 |

(Note 1) Sunlovely LFS-C (solid content 15%) (from Dohkai Chemical Industries Co., Ltd.)

<Preparation method>

(6), (10), (13), and (14) were added to (17) and the temperature was raised and adjusted to 70˚C. Next, the oil phase consisting of (1), (2), (3), (4), (5), (11), (12), (15), and (16) were prepared and the temperature was adjusted to 70˚C. This is added to the water phase previously prepared; a homomixer was used to homogenize the emulsified particles and (7), (8), (9), and (18) were added. After deaeration, filtration, and cooling, the target cream for wrinkle reduction was obtained.

[Example 1-15] Skin treatment composition for wrinkle reduction (lotion type)

| | Ingredients | Blend ratio (wt%) |
|---|---|---|
| (1) | Stearic acid | 2.0 |
| (2) | Cetyl alcohol | 1.5 |
| (3) | Petrolatum | 4.0 |

(continued)

| | Ingredients | Blend ratio (wt%) |
|---|---|---|
| (4) | Squalane | 5.0 |
| (5) | Glycerol tri-2-ethylhexanoate | 2.0 |
| (6) | Sorbitan monooleate | 2.0 |
| (7) | Dipropylene glycol | 5.0 |
| (8) | PEG 1500 | 3.0 |
| (9) | Water dispersion of polyurethane from preparation example 1 | 2.0 |
| (10) | Water dispersion of polyurethane from preparation example 2 | 8.0 |
| (11) | Water dispersion of an acrylic type polymer from preparation example 4 | 10.0 |
| (12) | Triethanolamine | 1. 0 |
| (13) | Methylparaben | 0. 1 |
| (14) | Phenoxy ethanol | 0. 1 |
| (15) | Perfume | 0. 1 |
| (16) | Purified water | Balance |
| | | Total 100.0 |

<Preparation method>

(7), (8), (12), (13), and (14) were added to (16) and the temperature was raised and adjusted to 70˚C. The oil phase consisting of (1), (2), (3), (4), (5), (6), and (15) were prepared and the temperature was adjusted to 70˚C. This oil phase was added to the water phase previously prepared and pre-emulsification was carried out. After homogenizing the emulsified particles with a homomixer, (9), (10), and (11) were added to obtain the target lotion for wrinkle reduction.

[Example 1-16] Skin treatment composition for wrinkle reduction (gel type)

| | Ingredients | Blend ratio (wt%) |
|---|---|---|
| (1) | Dipropylene glycol | 7.0 |
| (2) | PEG 1500 | 8.0 |
| (3) | Carboxyvinyl polymer | 0.4 |
| (4) | Methylcellulose | 0.2 |
| (5) | Polyoxyethylene (15) oleyl alcohol ether | 1. 0 |
| (6) | Potassium hydroxide | 0. 1 |
| (7) | Water dispersion of polyurethane from preparation example 1 | 3.0 |
| (8) | Water dispersion of polyurethane from preparation example 2 | 4.0 |
| (9) | Water dispersion of an acrylic type polymer from preparation example 3 | 5.0 |
| (10) | Edetate | 0.01 |
| (11) | Perfume | 0.1 |
| (12) | Purified water | Balance |
| (13) | Ethylparaben | 0.2 |
| | | Total 100.0 |

<Preparation method>

(3) and (4) were homogeneously dissolved in (12), to which (2) and (10) were added. (5) was added to (1) and heated and dissolved at 55˚C, to which (11) and (13) were added. This was slowly added to the water phase prepared previously as the latter was being stirred. Next, (7), (8), and (9) were added. Finally, an aqueous solution of (6) was added and thorough stirring was done for neutralization to obtain the target gel for wrinkle reduction.

[Example 1-17] Skin treatment composition for wrinkle reduction (lotion type)

| | Ingredients | Blend ratio (wt%) |
|---|---|---|
| (1) | Stearic acid | 2.0 |

(continued)

| | Ingredients | Blend ratio (wt%) |
|---|---|---|
| (2) | Cetyl alcohol | 1.5 |
| (3) | Petrolatum | 4.0 |
| (4) | Squalane | 5.0 |
| (5) | Glycerol tri-2-ethylhexanoate | 2.0 |
| (6) | Sorbitan monooleate | 2.0 |
| (7) | Dipropylene glycol | 5.0 |
| (8) | PEG 1500 | 3.0 |
| (9) | Water dispersion of polyurethane from preparation example 1 | 0.5 |
| (10) | Water dispersion of polyurethane from preparation example 2 | 9.5 |
| (11) | Water dispersion of an acrylic type polymer from preparation example 3 | 10.0 |
| (12) | Water dispersion of cross linked sodium poly-γ-glutamate (Note 1) | 10.0 |
| (13) | Triethanolamine | 1.0 |
| (14) | Methylparaben | 0. 1 |
| (15) | Phenoxy ethanol | 0. 1 |
| (16) | Perfume | 0.1 |
| (17) | Purified water | Balance |
| | | Total 100.0 |

(Note 1) Gelprotein A-8001 (solid content 2 wt%) (from Idemitsu Technofine Co., Ltd.)

<Preparation method>

(7), (8), (12), (13), (14), and (15) were added to (17) and the temperature was raised and adjusted to 70˚C. Next, the oil phase consisting of (1), (2), (3), (4), (5), (6), and (16) were prepared and the temperature was adjusted to 70˚C. This oil phase was added to the water phase prepared as above and pre-emulsification was carried out. After the emulsified particles were homogenized with a homomixer, (9), (10), and (11) were added to obtain the target lotion type for wrinkle reduction.

[Example 1-18] Skin treatment composition for wrinkle reduction (gel type)

| | Ingredients | Blend ratio (wt%) |
|---|---|---|
| (1) | Dipropylene glycol | 5.0 |
| (2) | Glycerin | 2.0 |
| (3) | PEG 1500 | 8.0 |
| (4) | Carboxyvinyl polymer | 0.4 |
| (5) | Methylcellulose | 0.2 |
| (6) | Polyoxyethylene (15) oleyl alcohol ether | 1. 0 |
| (7) | Potassium hydroxide | 0. 1 |
| (8) | Water dispersion of polyurethane from preparation example 1 | 1. 0 |
| (9) | Water dispersion of polyurethane from preparation example 2 | 6.0 |
| (10) | Water dispersion of an acrylic type polymer from preparation example 3 | 5.0 |
| (11) | Water dispersion of cross linked sodium poly-γ-glutamate (Note 1) | 50. 0 |
| (12) | Edetate | 0.01 |
| (13) | Perfume | 0.1 |
| (14) | Purified water | Balance |
| (15) | Ethylparaben | 0.2 Total 100.0 |

(Note 1) Gelprotein A-8001 (solid content 2 wt%) (from Idemitsu Technofine Co., Ltd.)

<Preparation method>

(4) and (5) were homogeneously dissolved in (14) and then (2), (3), (11), and (12) were added. (6) was added to (1) and heated and dissolved at 60˚C, to which (13) and (15) were added. This was slowly added to the water phase prepared previously as the latter was being stirred. Next, (7), (8), and (10) were added. Finally, an aqueous solution of (7) was added and thorough stirring was done for neutralization to obtain the target gel for wrinkle reduction.

[Invention of claims 13-25]

The present invention is described in detail below by referring to Examples. The blend ratios are in wt% units. Before the description of Examples, the method for evaluating/measuring the film and the efficacy test method used in the present invention are described.

[Method for evaluating/measuring the film)

(1) Film shrinkage rate

A water dispersion of a solid equivalent 1 g of the polymer is poured into a 5 cm x 5 cm polyethylene mold such that the film thickness is approximately 0.5 mm and dried at a prescribed temperature (50˚C) for a prescribed amount of time (three days at room temperature) to obtain a film, and vertical height and horizontal length of this film are measured and used in the following formula to calculate the film shrinkage rate.

$$\text{Film shrinkage rate (\%)} = [(\text{Vertical measurement} \times \text{Horizontal measurement})/25] \times 100$$

(2) Measurement of the strength and elongation of the film

A water dispersion of a solid equivalent 1 g of the polymer was poured into a 5 cm x 5 cm polyethylene mold such that the film thickness is approximately 0.5 mm and dried at a prescribed temperature (50˚C) for a prescribed amount of time (three days at room temperature) to obtain a film, and this film was cut out using a dumbbell #3 for the measurement. The autograph function of "Tensile tester RTM-250" from Orientec Co., Ltd. was used to carry out the measurement of the strength and elongation at 20˚C and a cross head speed of 300 mm/min.

(3) Particle size measurement of the water dispersion

The water dispersion of the polymer was measured with a laser light scattering particle size distribution measuring apparatus from Otsuka Electronics Co., Ltd.

[Sensory test]

Each specimen was used by a panel of 10 specialists. The specialists assessed each specimen for usability and the sensation during use, including (1) the wrinkle reduction effect right after application, (2) the wrinkle reduction effect five hours after application, (3) peeling off the skin (right after application, three hours after application), (4) glossiness (skin greasy and shining), (5) no stickiness (right after application, three hours after application), and (6) no discomfort; the evaluation was conducted by using the following criteria.

"Evaluation criteria".

(1) Wrinkle reduction effect right after application

◎ : 8 or more felt a wrinkle reduction effect.
○ : 5-7 felt a wrinkle reduction effect.

△ : 3-4 felt a wrinkle reduction effect.
× : 2 or fewer felt a wrinkle reduction effect.

(2) Wrinkle reduction effect five hours after application

◎ : 8 or more felt a wrinkle reduction effect.
○ : 5-7 felt a wrinkle reduction effect.
△ : 3-4 felt a wrinkle reduction effect.
× : 2 or fewer felt a wrinkle reduction effect.

(3) Peeling off from the skin

◎ : 8 or more felt there was no peeling off.
○ : 5-7 felt there was no peeling off.
△ : 3-4 felt there was no peeling off.
× : 2 or fewer felt there was no peeling off.

(4) Glossiness

◎ : 8 or more felt there was no glossiness.
○ : 5-7 felt there was no glossiness.
△ : 3-4 felt there was no glossiness.
× : 2 or fewer felt there was no glossiness.

(5) No stickiness

◎ : 8 or more felt there was no stickiness.
○ : 5-7 felt there was no stickiness.
△ : 3-4 felt there was no stickiness.
× : 2 or fewer felt there was no stickiness.

(6) No discomfort

◎ : 8 or more felt there was no discomfort.
○ : 5-7 felt there was no discomfort.
△ : 3-4 felt there was no discomfort.
× : 2 or fewer felt there was no discomfort.

[Examples 2-1 to 2-8, Comparative examples 2-1 to 2-11]

Skin treatment compositions for wrinkle reduction were prepared by mixing ingredients with blend ratios (total blend ratio 100 wt%) according to the recipes shown in Tables 2-1 to 2-4. Also, the efficacy test was conducted on the skin treatment compositions for wrinkle reduction from the aforementioned Examples 2-1 to 2-8 and Comparative examples 2-1 to 2-11; the evaluation results are also shown in Tables 2-1 to 2-4.

[Table 2-1]

| | Example | | | |
|---|---|---|---|---|
| | 2-1 | 2-2 | 2-3 | 2-4 |
| Ion-exchanged water | Balance | Balance | Balance | Balance |
| Glycerin | 3.0 | 3.0 | 3.0 | 3.0 |
| Water dispersion of polyurethane of preparation example 1 | 0.25 | 2.5 | - | 12.5 |
| | (0.05) | (0.5) | | (2.5) |
| Water dispersion of polyurethane of preparation example 2 | 0.25 | 2.5 | 10.0 | 5.0 |
| | (0.05) | (0.5) | (2.0) | (1.0) |
| Swollen form of non-emulsifying cross-linked silicone (Note 1) | - | 40. 0 | - | 24.0 |
| | | (5.0) | | (3.0) |
| Swollen form of non-emulsifying cross-linked silicone (Note 2) | 20.0 | - | 5.0 | - |
| | (1.0) | | (0.25) | |
| Dimethyl silicone (20 mPa·s) | 1.0 | 1.0 | 1.0 | 1.0 |
| Sorbitan polyoxyethylene (10) monooleate (Note 6) | 0.5 | 0.5 | 0.5 | 0.5 |
| Water dispersion of cross-linked sod i um poly-γ-glutamate (Note 8) | 30.0 | 30.0 | 30.0 | 30.0 |
| | (0.6) | (0.6) | (0.6) | (0.6) |
| Paraben | 0.15 | 0.15 | 0.15 | 0.15 |
| Total | 100 | 100 | 100 | 100 |
| Wrinkle reduction effect right after application | ○ | ◎ | ○ | ◎ |
| Wrinkle reduction effect five hours after application | ○ | ○ | ○ | ◎ |
| Peeling | ○ | ◎ | ◎ | ◎ |
| Glossiness | ○ | ◎ | ◎ | ◎ |
| No stickiness | ○ | ◎ | ◎ | ◎ |
| No discomfort | ○ | ◎ | ◎ | ○ |
| (Numbers in parentheses indicate the solid content.) | | | | |

[Table 2-2]

| | Example | | | |
|---|---|---|---|---|
| | 2-5 | 2-6 | 2-7 | 2-8 |
| Ion-exchanged water | Balance | Balance | Balance | Balance |
| Glycerin | 3.0 | 3.0 | 3.0 | 3.0 |
| Water dispersion of polyurethane of preparat i on example 1 | 25.0 | 35.0 | 20.0 | 30.0 |
| | (5.0) | (7.0) | (4.0) | (6.0) |
| Water dispersion of polyurethane of preparation example 2 | - | - | 20.0 | 20.0 |
| | | | (4.0) | (4.0) |
| Swollen form of non-emulsifying cross-linked silicone (Note 1) | 10.0 | - | - | 40.0 |
| | (1.25) | | | (5.0) |

(continued)

| | Example | | | |
|---|---|---|---|---|
| | 2-5 | 2-6 | 2-7 | 2-8 |
| Swollen form of non-emulsifying cross-linked silicone (Note 2) | - | 30.0 | 20.0 | - |
| | | (1.5) | (1.0) | |
| Dimethyl silicone (20 mPa·s) | 1.0 | 1.0 | 1.0 | 1.0 |
| Sorbitan polyoxyethylene (10) monooleate (Note 6) | 0.5 | 0.5 | 0.5 | 0.5 |
| Water dispersion of cross-linked sod i um poly- γ-glutamate (Note 8) | 30.0 | 30.0 | 30.0 | 30.0 |
| | (0.6) | (0.6) | (0.6) | (0.6) |
| Paraben | 0.15 | 0.15 | 0.15 | 0.15 |
| Total | 100 | 100 | 100 | 100 |
| Wrinkle reduction effect right after application | ◎ | ◎ | ◎ | ◎ |
| Wrinkle reduction effect five hours after application | ○ | ○ | ○ | ◎ |
| Peeling | ◎ | ◎ | ◎ | ○ |
| Glossiness | ◎ | ◎ | ◎ | ○ |
| No stickiness | ◎ | ◎ | ◎ | ○ |
| No discomfort | ○ | ○ | ○ | ○ |
| (Numbers in parentheses indicate the solid content.) | | | | |

[Table 2-3]

| | Comparative example | | | | | |
|---|---|---|---|---|---|---|
| | 2-1 | 2-2 | 2-3 | 2-4 | 2-5 | 2-6 |
| Ion-exchanged water | Balance | Balance | Balance | Balance | Balance | Balance |
| Glycerin | 3.0 | 3.0 | 3.0 | 3.0 | 3.0 | 3.0 |
| Water dispersion of polyurethane of preparation example 1 | 2.5 (0.5) | 20.0 (4.0) | - | - | - | 35.0 (7.0) |
| Water dispersion of polyurethane of preparation example 2 | 2.5 (0.5) | 20.0 (4.0) | - | - | - | - |
| Swollen form of non-emulsifying cross-linked silicone (Note 1) | - | - | 10.0 (1.25) | - | 40.0 (5.0) | - |
| Dimethyl silicone (20 mPa·s) | 1.0 | 1.0 | 1.0 | 1.0 | 1.0 | 1.0 |
| Sorbitan polyoxyethylene (10) monooleate (Note 6) | 0.5 | 0.5 | 0.5 | 0.5 | 0.5 | 0.5 |
| Water dispersion of cross-linked sodium poly-γ-glutamate (Note 8) | 30.0 (0.6) | 30.0 (0.6) | 30.0 (0.6) | 30.0 (0.6) | 30.0 (0.6) | 30.0 (0.6) |
| Paraben | 0.15 | 0.15 | 0.15 | 0.15 | 0.15 | 0.15 |
| Total | 100 | 100 | 100 | 100 | 100 | 100 |
| Wrinkle reduction effect right after application | × | ○ | △ | × | ○ | ○ |
| Wrinkle reduction effect five hours after application | × | ○ | △ | × | ○ | △ |
| Peeling | × | × | ○ | × | × | × |
| Glossiness | △ | × | △ | ◎ | ◎ | △ |
| No stickiness | ○ | × | × | ◎ | × | × |
| No discomfort | ○ | × | ○ | × | △ | × |

(Numbers in parentheses indicate the solid content.)

[Table 2-4]

| | Comparative example | | | | |
|---|---|---|---|---|---|
| | 2-7 | 2-8 | 2-9 | 2-10 | 2-11 |
| Ion-exchanged water | Balance | Balance | Balance | Balance | Balance |
| Glycerin | 3.0 | 3.0 | 3.0 | 3.0 | 3.0 |
| Water dispersion of polyurethane of preparation example 1 | 10.0 | - | - | - | - |
| | (2.0) | | | | |
| Swollen form of non-emulsifying cross-linked silicone (Note 1) | - | - | 40.0 | - | - |
| | | | (5.0) | | |

(continued)

| | Comparative example | | | | |
|---|---|---|---|---|---|
| | 2-7 | 2-8 | 2-9 | 2-10 | 2-11 |
| Swollen form of non-emulsifying cross-linked silicone (Note 2) | - | - | - | 10.0 | - |
| | | | | (0.5) | |
| Water dispersion of polyurethane (Note 3) | - | - | 6.06 | - | - |
| | | | (2.0) | | |
| Dimethyl silicone (20 mPa·s) | - | - | - | - | 10.0 |
| Water dispersion of polyurethane (Note 4) | - | - | - | 25.0 | - |
| | | | | (5.0) | |
| Water dispersion of polyurethane (Note 5) | - | - | - | - | 15.0 |
| | | | | | (3.0) |
| Sorbitan polyoxyethylene (10) monooleate (Note 6) | 0.5 | 0.5 | 0.5 | 0.5 | 0.5 |
| Trimethylsiloxysilicic acid (Note 7) | 5.0 | - | - | - | - |
| | (2.5) | | | | |
| Polyvinyl alcohol | - | 3.0 | - | - | - |
| Water dispersion of cross-linked sodium poly-γ-glutamate (Note 8) | 30.0 | 30.0 | 30.0 | 30.0 | 30.0 |
| | (0.6) | (0.6) | (0.6) | (0.6) | (0.6) |
| Paraben | 0.15 | 0.15 | 0.15 | 0.15 | 0.15 |
| Total | 100 | 100 | 100 | 100 | 100 |
| Wrinkle reduction effect right after application | △ | × | × | △ | △ |
| Wrinkle reduction effect five hours after application | × | × | △ | × | △ |
| Peeling | △ | × | △ | × | △ |
| Glossiness | × | ○ | × | × | △ |
| No stickiness | ○ | × | ○ | × | × |
| No discomfort | × | ◎ | △ | × | △ |
| (Numbers in parentheses indicate the solid content.) | | | | | |

In Tables 2-1 to 2-4:

(Note 1) DC9045 (solid content 12.5%) (from Dow Corning Toray)
(Note 2) KSG-44 (solid content approximately 5%) (from Shin-Etsu Chemical Co., Ltd.)
(Note 3) Avalure UR405 (solid content 33%) (from NOVEON)
(Average particle size: 100 nm, strength: 40 kg/cm$^2$, elongation: 150%, film shrinkage rate: 35%)
(Note 4) Avalure UR445 (solid content 20%) (from NOVEON)
(Average particle size: 5 nm, strength: 240 kg/cm$^2$, elongation: 610%, film shrinkage rate: 27%)
(Note 5) Iodosol PUD (solid content 20%) (from Nippon NSC)
(Average particle size: 20 nm, strength: 200 kg/cm$^2$, elongation: 530%, film shrinkage rate: 23%)
(Note 6) NIKKOL TO-10 (from Nikko Chemicals Co.) (Note 7) Contains KF7312J (decamethylcyclopentasiloxane solution; effective ingredient content 50%) (from Shin-Etsu Chemical Co., Ltd.).
(Note 8) Gelprotein A-8001 (solid content 2 %) (from Idemitsu Technofine Co., Ltd.)

As clearly shown in Tables 2-1 to 2-4, the skin treatment compositions for wrinkle reduction from Examples 2-1 to 2-8 pertaining to the present invention are superior in terms of the wrinkle reduction effect and free of peeling, glossiness, stickiness, and discomfort. On the contrary, skin treatment compositions for wrinkle reduction from Comparative examples 2-1 to 2-11 which do not meet the constituent requirements of the present invention, do not manifest

the effects of the present invention.

More Examples of the skin treatment composition for wrinkle reduction of the present invention are described below. The aforementioned efficacy test was conducted on these and the results were superior for all of them.

[Example 2-9] Skin treatment composition for wrinkle reduction (cream type)

| Ingredients | | Blend ratio (wt%) |
|---|---|---|
| (1) | Stearyl alcohol | 6.0 |
| (2) | Stearic acid | 2.0 |
| (3) | Hydrated lanolin | 4.0 |
| (4) | Squalane | 9.0 |
| (5) | 5-octyldodecanol | 10.0 |
| (6) | 1,3-butylene glycol | 6.0 |
| (7) | Water dispersion of polyurethane from preparation example 1 | 5.0 (solid content 1. 0) |
| (8) | Water dispersion of polyurethane from preparation example 2 | 10.0 (solid content 2) |
| (9) | Swollen form of non-emulsifying cross-linked silicone (Note 1) | 20.0 (solid content 3.2) |
| (10) | PEG 1500 | 4.0 |
| (11) | Polyoxyethylene (20) sorbitan monococoate (Note 2) | 3. 0 |
| (12) | Glycerin monostearate | 2.0 |
| (13) | Ethylparaben | 0.1 |
| (14) | Butylparaben | 0.1 |
| (15) | Tocopherol | 0.01 |
| (16) | Perfume | 0.1 |
| (17) | Purified water | Balance |
| | | Total 100.0 |

(Note 1) DC9041 (solid content 16%) (from Dow Corning Toray)
(Note 2) NIKKOL TL-10 (from Nikko Chemicals Co.)

<Preparation method>

(6), (10), (13), and (14) were added to (17) and the temperature was raised and adjusted to 70˚C. Next, the oil phase consisting of (1), (2), (3), (4), (5), (9), (11), (12), (15), and (16) were prepared and the temperature was adjusted to 70˚C. This was added to the water phase previously prepared; a homomixer was used to homogenize the emulsified particles and (7) and (8) were added. After deaeration, filtration, and cooling, the target cream for wrinkle reduction was obtained.

[Example 2-10] Skin treatment composition for wrinkle reduction (lotion type)

| Ingredients | | Blend ratio (wt%) |
|---|---|---|
| (1) | Palmitic acid | 2.0 |
| (2) | Cetyl alcohol | 1.5 |
| (3) | Petrolatum | 4.0 |
| (4) | Squalane | 5.0 |
| (5) | Glycerol tri-2-ethylhexanoate | 2.0 |
| (6) | Sorbitan monooleate | 2.0 |
| (7) | Dipropylene glycol | 5.0 |
| (8) | PEG 1500 | 3.0 |
| (9) | Water dispersion of polyurethane from preparation example 1 | 2.0 (solid content 0.4) |
| (10) | Water dispersion of polyurethane from preparation example 2 | 8.0 (solid content 1.6) |

(continued)

| | Ingredients | Blend ratio (wt%) |
|---|---|---|
| (11) | Swollen form of non-emulsifying cross-linked silicone (Note 1) | 30.0 (solid content 1.8) |
| (12) | Triethanolamine | 1.0 |
| (13) | Methylparaben | 0.1 |
| (14) | Phenoxy ethanol | 0. 1 |
| (15) | Perfume | 0. 1 |
| (16) | Purified water | Balance |
| | | Total 100.0 |

(Note 1) GRANSIL GCM-5 (solid content approximately 6%) (from GRANT)

<Preparation method>

(7), (8), (13), and (14) were added to (16) and the temperature was raised and adjusted to 70˚C. The oil phase consisting of (1), (2), (3), (4), (5), (6), (11), and (15) were prepared and the temperature was adjusted to 70˚C. This oil phase was added to the water phase prepared previously and pre-emulsification was carried out. Next, (12) was added; after the emulsified particles were homogenized with a homomixer, (9) and (10) were added to obtain the target lotion type for wrinkle reduction.

[Example 2-11] Skin treatment composition for wrinkle reduction (cream type)

| | Ingredients | Blend ratio (wt%) |
|---|---|---|
| (1) | Behenyl alcohol | 1.0 |
| (2) | Batyl alcohol | 2.0 |
| (3) | Hydrated polyisobutene | 4.0 |
| (4) | Liquid petrolatum | 9.0 |
| (5) | Decamethylcyclopentasiloxane | 10.0 |
| (6) | 1,3-butylene glycol | 3.0 |
| (7) | Glycerin | 5.0 |
| (8) | Water dispersion of polyurethane from preparation example 1 | 5.0 (solid content 1.0) |
| (9) | Water dispersion of polyurethane from preparation example 2 | 10.0 (solid content 2.0) |
| (10) | Swollen form of non-emulsifying cross-linked silicone (Note 1) | 20.0 (solid content 3.0) |
| (11) | PEG 20000 | 4.0 |
| (12) | Self emulsified glycerin monostearate (Note 2) | 3. 0 |
| (13) | Glycerin monostearate | 2.0 |
| (14) | Ethylparaben | 0. 1 |
| (15) | Butylparaben | 0. 1 |
| (16) | Tocopherol | 0.01 |
| (17) | Perfume | 0. 1 |
| (18) | Purified water | Balance |
| | | Total 100.0 |

<Preparation method>

(6), (7), (11), (14), and (15) were added to (18) and the temperature was raised and adjusted to 70˚C. Next, the oil phase consisting of (1), (2), (3), (4), (5), (10), (12), (15), (16), and (17) were prepared and the temperature was adjusted to 70˚C. This was added to the water phase previously prepared; a homomixer was used to homogenize the emulsified particles and (8) and (9) were added. After deaeration, filtration, and cooling, the target cream for wrinkle reduction was obtained.

(Note 1) GRANSIL ININ (solid content approximately 15%) (from GRANT)

(Note 2) NIKKOL MGS-ASE (from Nikko Chemicals Co.)

[Example 2-12] Skin treatment composition for wrinkle reduction (lotion type)

| Ingredients | | Blend ratio (wt%) |
|---|---|---|
| (1) | Palmitic acid | 2.0 |
| (2) | Cetyl alcohol | 1.5 |
| (3) | Petrolatum | 4.0 |
| (4) | Squalane | 5.0 |
| (5) | Glycerol tri-2-ethylhexanoate | 2.0 |
| (6) | Sorbitan monooleate | 2.0 |
| (7) | Dipropylene glycol | 5.0 |
| (8) | PEG 1500 | 3.0 |
| (9) | Water dispersion of polyurethane from preparation example 1 | 2.0 (solid content 0.4) |
| (10) | Water dispersion of polyurethane from preparation example 2 | 8.0 (solid content 1.6) |
| (11) | Swollen form of non-emulsifying cross-linked silicone (Note 1) | 30.0 (solid content 1.5) |
| (12) | Triethanolamine | 1.0 |
| (13) | Methylparaben | 0.1 |
| (14) | Phenoxy ethanol | 0.1 |
| (15) | Perfume | 0.1 |
| (16) | Purified water | Balance |
| | | Total 100.0 |

(Note 1) KSG-44 (solid content approximately 5%) (from Shin-Etsu Chemical Co., Ltd.)

<Preparation method>

(7), (8), (13), and (14) were added to (16) and the temperature was raised and adjusted to 70˚C. The oil phase consisting of (1), (2), (3), (4), (5), (6), (11), and (15) were prepared and the temperature was adjusted to 70˚C. This oil phase was added to the water phase prepared previously and pre-emulsification was carried out. Next, (12) was added; after the emulsified particles were homogenized with a homomixer, (9) and (10) were added to obtain the target lotion type for wrinkle reduction.

[Example 2-13] Skin treatment composition for wrinkle reduction (gel type)

| Ingredients | | Blend ratio (wt%) |
|---|---|---|
| (1) | Copolymer of sodium polyacrylate/2-acrylamide-2-methylpropanesulfonic acid (AMPS) (Note 1) | 2. 1 (effective ingredient content 0.79) |
| (2) | Water dispersion of polyurethane from preparation example 1 | 2.0 (solid content 0.4) |
| (3) | Water dispersion of polyurethane from preparation example 2 | 8.0 (solid content 1.6) |
| (4) | Swollen form of non-emulsifying cross-linked silicone (Note 2) | 30.0 (solid content 1. 5) |
| (5) | Triethanolamine | 1. 0 |
| (6) | Methylparaben | 0. 1 |
| (7) | Phenoxy ethanol | 0. 1 |
| (8) | Perfume | 0. 1 |
| (9) | Purified water | Balance |
| (10) | Glycerin | 5.0 |

(continued)

| Ingredients | | Blend ratio (wt%) |
|---|---|---|
| (11) | 1,3-butylene glycol | 3.0 |
| | | Total 100.0 |

(Note 1) SIMULGE EG (effective ingredient content 37.5%) (from Sepic)
(Note 2) KSG-15 (solid content approximately 5%) (from Shin-Etsu Chemical Co., Ltd.)

<Preparation method>

(1), (5), (6), (7), (10), and (11) were added to (9), to which a mixture of (4) and (8) were added and dispersed homogeneously with a homomixer. (2) and (3) were then added to obtain the target gel for wrinkle reduction.

[Example 2-14] Skin treatment composition for wrinkle reduction (cream type)

| Ingredients | | Blend ratio (wt%) |
|---|---|---|
| (1) | Behenyl alcohol | 2.0 |
| (2) | Stearyl alcohol | 3.0 |
| (3) | Octyl palmitate | 3.0 |
| (4) | $\alpha$-olefin oligomer | 5. 0 |
| (5) | Dimethylpolysiloxane (6 mPa·s) | 10.0 |
| (6) | 1,3-butylene glycol | 3.0 |
| (7) | Glycerin | 5.0 |
| (8) | Water dispersion of polyurethane from preparation example 1 | 5 (solid content 1) |
| (9) | Water dispersion of polyurethane from preparation example 2 | 10.0 (solid content 2.0) |
| (10) | Swollen form of non-emulsifying cross-linked silicone (Note 1) | 20.0 (solid content 5) |
| (11) | PEG 20000 | 4.0 |
| (12) | Polyoxyethylene (20) sorbitan monococoate (Note 2) | 3. 0 |
| (13) | Glycerin monostearate | 2.0 |
| (14) | Ethylparaben | 0.1 |
| (15) | Butylparaben | 0.1 |
| (16) | Tocopherol | 0.01 |
| (17) | Perfume | 0.1 |
| (18) | Purified water | Balance |
| | | Total 100.0 |

(Note 1) KSG-16 (solid content approximately 25%) (from Shin-Etsu Chemical Co., Ltd.)
(Note 2) NIKKOL TL-10 (from Nikko Chemicals Co.)

<Preparation method>

(6), (7), (11), (14), and (15) were added to (18) and the temperature was raised and adjusted to 70˚C. Next, the oil phase consisting of (1), (2), (3), (4), (5), (10), (12), (15), (16), and (17) were prepared and the temperature was adjusted to 70˚C. This was added to the water phase previously prepared; a homomixer was used to homogenize the emulsified particles and (8) and (9) were added. After deaeration, filtration, and cooling, the target cream for wrinkle reduction was obtained.

## Claims

1. A skin treatment composition having a water dispersion of a polymer in which a non-water soluble film-forming polymer is dispersed in water wherein the main ingredients of said film-forming polymer are polyurethane having a

film shrinkage rate of 20% or less and an acrylic type polymer having a film shrinkage rate of 20% or less.

2. The skin treatment composition for wrinkle reduction of claim 1 wherein said polyurethane is polyurethane obtained by reacting an isocyanate compound and a diol compound containing a polyether diol, polycarbonate diol, and alkylene diol containing a carboxyl group.

3. The skin treatment composition for wrinkle reduction of claim 2 comprising isophorone diisocyanate for the isocyanate compound.

4. The skin treatment composition for wrinkle reduction of claim 2 or 3 wherein the polyether diol is polytetramethylene glycol, the polycarbonate diol is polyhexamethylene carbonate diol, and the alkylene diol containing a carboxyl group is dimethylolpropionic acid and/or dimethylolbutanoic acid.

5. The skin treatment composition for wrinkle reduction of one of claims 1 to 4 wherein the acrylic type polymer is a polymer from monomers whose main ingredient is ethyl acrylate.

6. The skin treatment composition for wrinkle reduction of one of claims 1 to 5 wherein the water dispersion of an acrylic type polymer contains sulfonated polyvinyl alcohol.

7. The skin treatment composition for wrinkle reduction of one of claims 1 to 6 wherein the film strength of the polyurethane is 300-700 kg/cm$^2$ and the film strength of the acrylic type polymer is 0.1-100 kg/cm$^2$.

8. The skin treatment composition for wrinkle reduction of one of claims 1 to 7 wherein the film elongation of the polyurethane is 200-500%, and the film elongation of the acrylic type polymer is 500-2000%.

9. The skin treatment composition for wrinkle reduction of one of claims 1 to 8 wherein the average particle size of the polyurethane in the water dispersion of polyurethane is 10-300 nm, and the average particle size of the acrylic type polymer in the water dispersion of an acrylic type polymer is 100-600 nm.

10. The skin treatment composition for wrinkle reduction of one of claims 1 to 9 wherein the polyurethane in the water dispersion of polyurethane is a mixture of particles having an average particle size of 20-60 nm and particles having an average particle size of 150-200 nm.

11. The skin treatment composition for wrinkle reduction of one of claims 1 to 10 that contains the polyurethane to 1-10 wt% of the total weight of the skin treatment composition and the acrylic type polymer to 1-20 wt% of the total weight of the skin treatment composition.

12. The skin treatment composition for wrinkle reduction of one of claims 1 to 11 that further comprises scaly silica.

13. A skin treatment composition for wrinkle reduction comprising (a) a non-emulsification type cross-linked silicone, (b) a film forming polymer having a film shrinkage rate of 20% or less containing as a main ingredient a polyurethane having a film shrinkage rate of 20%, (c) a liquid oil component, and (d) water.

14. The skin treatment composition for wrinkle reduction of claim 13 wherein (a) the non-emulsification type cross-linked silicone is one, two, or more chosen from a group consisting of a cross polymer derived from a reaction between methyl hydrogen polysiloxane and methyl vinyl polysiloxane, a cross polymer derived from a reaction between a partial long chain alkylated methyl hydrogen polysiloxane and methyl vinyl polysiloxane, and a cross polymer derived from a reaction between methyl hydrogen polysiloxane and alkene.

15. The skin treatment composition for wrinkle reduction of claim 14 wherein the number of carbons in the long chain alkyl in the partial long chain alkylated methyl hydrogen polysiloxane is 10-14.

16. The skin treatment composition for wrinkle reduction of one of claims 13 to 15 wherein the non-emulsion type cross-linked silicone is added as it is swollen with a liquid oil component.

17. The skin treatment composition for wrinkle reduction of one of claims 13 to 16 wherein said polyurethane is polyurethane obtained by reacting an isocyanate compound and a diol compound containing a polyether diol, polycarbonate diol, and alkylene diol containing a carboxyl group.

**18.** The skin treatment composition for wrinkle reduction of claim 17 comprising isophorone diisocyanate for the isocyanate compound.

**19.** The skin treatment composition for wrinkle reduction of claim 17 or 18 wherein the polyether diol is polytetramethylene glycol, the polycarbonate diol is polyhexamethylene carbonate diol, and the alkylene diol containing a carboxyl group is dimethylolpropionic acid and/or dimethylolbutanoic acid.

**20.** The skin treatment composition for wrinkle reduction of one of claims 13 to 19 wherein the film strength of the polyurethane is 300-700 kg/cm$^2$.

**21.** The skin treatment composition for wrinkle reduction of one of claims 13 to 20 wherein the film elongation of the polyurethane is 200-500%.

**22.** The skin treatment composition for wrinkle reduction of one of claims 13 to 21 wherein a film forming polymer having a film shrinkage rate of 20% or less containing as a main ingredient a polyurethane having a film shrinkage rate of 20% is added in the form of a water dispersion.

**23.** The skin treatment composition for wrinkle reduction of claim 22 wherein the average particle size of the polyurethane in the water dispersion of polyurethane is 10-300 nm.

**24.** The skin treatment composition for wrinkle reduction of one of claims 22 to 23 wherein the polyurethane in the water dispersion of polyurethane is a mixture of particles having an average particle size of 20-60 nm and particles having an average particle size of 150-200 nm.

**25.** The skin treatment composition for wrinkle reduction of one of claims 13 to 24 that contains the non-emulsion type cross-linked silicone to 0.5-5.0 wt% of the total amount of the skin treatment composition and the polyurethane having a film shrinkage rate of 20% or less to 0.1-10.0 wt% of the total amount of the skin treatment composition

**INTERNATIONAL SEARCH REPORT**

International application No.
PCT/JP2005/000332

A. CLASSIFICATION OF SUBJECT MATTER
Int.Cl[7] A61K7/48, A61K7/00, C08L75/04, C08G18/66

According to International Patent Classification (IPC) or to both national classification and IPC

B. FIELDS SEARCHED

Minimum documentation searched (classification system followed by classification symbols)
Int.Cl[7] A61K7/48, A61K7/00, C08L75/04, C08G18/66

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 11-507396 A (L'Oreal),<br>29 June, 1999 (29.06.99),<br>Claims<br>& AT 175109 T & BR 9707855 A<br>& CA 2199053 A1 & CN 1212617 A<br>& DE 69700082 T & EP 0793957 B1<br>& ES 2129998 T & FR 2745494 B1<br>& JP 2004-043500 A & PL 328600 A1<br>& US 6010686 A & WO 97/32566 A1 | 1-11<br>12-25 |
| Y | JP 06-087720 A (Nippon Sheet Glass Co., Ltd.),<br>29 March, 1994 (29.03.94),<br>Abstract<br>(Family: none) | 12 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 April, 2005 (11.04.05) | 10 May, 2005 (10.05.05) |
| Name and mailing address of the ISA/<br>Japanese Patent Office<br><br>Facsimile No. | Authorized officer<br><br>Telephone No. |

Form PCT/ISA/210 (second sheet) (January 2004)

**INTERNATIONAL SEARCH REPORT**

International application No.
PCT/JP2005/000332

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2001-294510 A (Avon Products,Inc.),<br>23 October, 2001 (23.10.01),<br>Claims 1, 14, 15, 17, 19<br>& AU 774676 B2 & AU 4004601 A1<br>& BR 008214 A & BR 0008214 A<br>& BR 0109074 A & CA 2341120 A1<br>& CA 2364300 A1 & CA 2397183 A1<br>& CA 2413919 A1 & CN 1364073 A<br>& CN 1411360 A & EP 1136064 A2<br>& EP 1152731 A1 & EP 1261314 A1<br>& EP 1265583 A1 & JP 2003-516950 A<br>& MXPA 02008725 A & PL 349174 A1<br>& US 2002/006383 A1 | 13-22,25<br>13-25 |
| A | JP 2003-321326 A (L'Oreal),<br>11 November, 2003 (11.11.03),<br>& AT 280568 A & DE 60300114 D<br>& EP 1352626 A1 & FR 2838343 A1<br>& US 2003/215476 A1 | 1-25 |
| A | JP 11-504949 A (L'Oreal),<br>11 May, 1999 (11.05.99),<br>& AU 5770298 A & EP 0944381 A1<br>& FR 2758084 B1 & WO 98/29092 A1 | 1-25 |
| E,A | JP 2004-161771 A (L'Oreal),<br>10 June, 2004 (10.06.04),<br>& EP 1419763 A1 & FR 2846884 A1<br>& US 2004/136937 A1 | 1-25 |
| E,A | JP 2004-168667 A (Dokai Kagaku Kogyo<br>Kabushiki Kaisha),<br>17 June, 2004 (17.06.04),<br>(Family: none) | 12 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11504949 A
- JP H5933 A